# EUROPÄISCHE PATENTSCHRIFT

(11) **EP 1 727 829 B1**
(45) Veröffentlichungstag und Bekanntmachung des Hinweises auf die Patenterteilung: **15.02.2012**
(21) Anmeldenummer: 05716078.0
(22) Anmeldetag: 15.03.2005
(51) Int. Cl.: C07K 14/40

(54) **HYPHENSPEZIFISCHE ZELLWANDPROTEINE VON CANDIDA**
HYPHA-SPECIFIC CELL WALL PROTEINS OF CANDIDA
PROTEINES DE PAROI CELLULAIRE DE CANDIDA, SPECIFIQUES DE L'HYPHE

(30) Priorität: 16.03.2004 DE 102004013826
(43) Veröffentlichungstag der Anmeldung: 06.12.2006
(73) Patentinhaber: Fraunhofer-Gesellschaft zur Förderung der angewandten Forschung e.V., 80686 München (DE)
(72) Erfinder: LOTZ, Henrike, 70176 Stuttgart (DE); BRUNNER, Herwig, 70569 Stuttgart (DE); RUPP, Steffen, 70569 Stuttgart (DE)
(74) Vertreter: Schwahn, Hartmut
(86) Internationale Anmeldenummer: PCT/EP2005/002748
(87) Internationale Veröffentlichungsnummer: WO 2005/090397

(56) Entgegenhaltungen:
- US-B1- 6 747 137
- SOHN K., ET AL: "EFG1 is a major regulator of cell wall dynamics in Candida Albicans as revealed by DNA microarrays" MOLECULAR MICRONIOLOGY, Bd. 47, Nr. 1, 2003, Seiten 89-102, XP002331978 in der Anmeldung erwähnt
- KONDORI N ET AL: "CANDIDA ALBICANS CELL WALL ANTIGENS FOR SEROLOGICAL DIAGNOSIS OF CANDIDEMIA" MEDICAL MYCOLOGY, OXFORD, GB, Bd. 41, Nr. 1, Februar 2003 (2003-02), Seiten 21-30, XP008025341 ISSN: 1369-3786

## Beschreibung

Die vorliegende Erfindung betrifft Nucleinsäuren, die ein zur Hyphenentwicklung von *Candida* erforderliches Zellwandprotein codieren, Fragmente der Nucleinsäuren, die Nucleinsäuren oder Nucleinsäure-Fragmente enthaltende Vektoren, die Vektoren enthaltende Wirtszellen, die von den Nucleinsäuren codierten Zellwandproteine, gegen die Proteine gerichtete Antikörper, Verfahren zur Herstellung der Zellwandproteine, Verfahren zur Charakterisierung und/oder zum Nachweis des Hyphenstadiums von *Candida,* Verfahren zum Nachweis einer *Candida*-Infektion, Verfahren zum Identifizieren von Substanzen mit therapeutischer Wirkung gegen durch *Candida* verursachte Krankheiten, diagnostische und pharmazeutische Zusammensetzungen, die die Nucleinsäuren, Nucleinsäure-Fragmente, Vektoren, Wirtszellen, Proteine und/oder Antikörper gegen das Zellwandprotein enthalten, Kits zur in vitro-Identifizierung der Zellwandproteine sowie die Verwendung der Nucleinsäuren, Vektoren, Proteine oder Antikörper zur Diagnose und Behandlung von *Candida*verursachten Krankheiten beziehungsweise zur Herstellung von diagnostischen oder pharmazeutischen Zusammensetzungen.

*Candida albicans* ist der häufigste Erreger von systemischen Mykosen beim Menschen. Normalerweise besiedelt *C*. *albicans* als Kommensale die Oberflächen des Gastrointestinal-Traktes und der Schleimhäute von Mensch und Tier. Während einer systemischen Infektion kann *C*. *albicans* jedoch eine Vielzahl wichtiger Organe eines Organismus befallen. Bei immunsupprimierten Patienten kann *C*. *albicans* häufig opportunistische Infektionen verursachen. Insbesondere bei immunsupprimierten Patienten können *Candida-*Infektionen einen äußerst schweren Verlauf nehmen und zu lebensbedrohenden Zuständen führen. Die Inzidenz invasiver und schwer zu behandelnder *Candida*-Mykosen nimmt weltweit deutlich zu. Zu den wichtigsten Risikofaktoren für die Entwicklung von Pilzinfektionen wie *Candida*-Mykosen gehören Neutropenie, aggressive Tumortherapie, Immunsuppression, AIDS, Antibiotika-Therapien, Diabetes mellitus, eine sehr lange Verweildauer intravenöser Katheder, Graftversus-Host-Krankheit und chirurgische Eingriffe.

Die exakte Diagnose pilzlicher Infektionen, insbesondere von *Candida*-Infektionen, ist äußerst schwierig, da die klinischen Befunde bei Pilzinfektionen überwiegend uncharakteristisch sind. Zur Diagnostik invasiver Pilzinfektionen werden bisher hauptsächlich Kulturen zur exakten Spezies-Bestimmung angelegt, wobei jedoch auf Kulturen basierende diagnostische Tests im Allgemeinen nicht ausreichend genau und sensitiv sind. Darüber hinaus ist es auch sehr schwer, eine Unterscheidung zwischen einer Kolonisierung und einer Invasion des Pilz-Pathogenes vorzunehmen. Zur Diagnose von Pilzinfektionen werden auch Proben des Patienten mikroskopisch untersucht. Vor allem bei Vorliegen von nur wenigen Hyphenfragmenten ist allerdings eine Spezies-Unterscheidung und daher auch eine genaue Spezies-Bestimmung nicht immer möglich. Zum Nachweis invasiver Pilzinfektionen, insbesondere *Candida*-Infektionen, werden auch serologische Verfahren eingesetzt, wobei sich jedoch hier herausgestellt hat, dass die derzeit verfügbaren Antikörper-Tests bei immunsupprimierten Patienten teilweise nur abgeschwächt reagieren, was eine Interpretation der Titer-Kinetik erschwert. In den letzten Jahren wurden darüber hinaus Verfahren zum Nachweis von Pilzspezifischer DNA mit Hilfe von Hybridisierungsverfahren und der Polymerase-Kettenreaktion (PCR) entwickelt, die jedoch in der klinischen Anwendung eine zu geringe Sensitivität und Spezifität zeigen. Problematisch ist insbesondere, dass die derzeit eingesetzten DNA-Extraktions- und Amplifikationsverfahren kein Routine-Screening von Patienten erlauben. Sohn et al. (Sohn et al., Mol. Microbiol., 47 (2003), 89-102) beschreiben DNA Sequenzen von C. albicans, die Hyphen-Zellwandproteine codieren. Insgesamt zeigt sich, dass derzeit eine zuverlässige Diagnose von *Candida*-Infektionen sehr schwer ist, da die Diagnose-Verfahren nicht ausreichend sensitiv oder spezifisch sind, wobei invasive Pilz-Infektionen zumeist erst relativ spät erfasst werden können.

Die klinische Behandlung von *C*. *albicans*-Infektionen erfolgt derzeit hauptsächlich unter Verwendung von Polyen-Antibiotika, Azol-Derivaten, Alylaminen/Thiocarbamaten, Fluoropyrimidinen sowie E-chinocandinen. Die derzeit verwendeten Antimykotika tragen den medizinischen Erfordernissen jedoch nur teilweise Rechnung. Azole, beispielsweise Ketoconazol, sind hochtoxisch und weisen eine Reihe unerwünschte Nebenwirkungen auf. Auch hat sich gezeigt, dass sich in zunehmendem Maße gegen die eingesetzten Azole und Echinocandine Resistenzen entwickeln (Schuetzer-Muehlbauer et al., Mol. Microbiol., 48 (2003), 223-235). Polyen-Makrolide wie Amphothericin B und Nystatin führen aufgrund ihrer hohen Toxizität ebenfalls zu starken Nebenwirkungen.

Die Entwicklung weiterer verbesserter Diagnostika, die eine zuverlässige Zuordnung einer Erkrankung zu den durch die Gattung *Candida* hervorgerufenen Infektionen gestatten, und weiterer verbesserter Antimykotika zur Behandlung von Infektionen oder Krankheiten die durch *Candida* hervorgerufen werden, ist daher dringend erforderlich.

Das der vorliegenden Erfindung zugrundeliegende technische Problem besteht daher in der Bereitstellung von Mitteln und Verfahren, die eine exakte und schnelle Diagnose der von *Candida,* insbesondere *C*. *albicans* hervorgerufenen Krankheiten und Infektionen ermöglichen, sowie in der Bereitstellung von Mitteln und Verfahren, die zur Entwicklung neuartiger hochwirksamer Therapeutika eingesetzt werden können, die insbesondere eine gezielte Therapie von durch *Candida* hervorgerufenen Infektionen ermöglichen und die nicht die im Stand der Technik bekannten Nachteile aufweisen, beispielsweise keine Nebenwirkungen im behandelten Organismus hervorrufen.

Die Erfindung löst das ihr zugrundeliegende technische Problem durch die Bereitstellung des Gegenstands nach Anspruch 1 sowie der Gegenstände der Nebenansprüche und Unteransprüche.

Offenbart ist die Bereitstellung eines Nucleinsäuremoleküls, das ein zur Hyphenentwicklung eines pathogenen pilzlichen Organismus erforderliches Zellwandprotein codiert, ausgewählt aus der Gruppe bestehend aus:
a) einem Nucleinsäuremolekül mit einer der in SEQ ID Nr. 1, SEQ ID Nr. 3 oder SEQ ID Nr. 5 dargestellten Nucleodidsequenzen,
b) einem Nucleinsäuremolekül mit einer Nucleotidsequenz, die ein Protein mit einer der in SEQ ID Nr. 2, SEQ ID Nr. 4 oder SEQ ID Nr. 6 dargestellten Aminosäuresequenzen codiert,
c) einem Nucleinsäuremolekül mit einer Nucleotidsequenz, die über ihre gesamte Länge eine Homologie von mindestens 80 % zu einer Nucleotidsequenz eines der Nucleinsäuremoleküle von a) oder b) zeigt, und
d) einem Nucleinsäuremolekül mit einer Nucleotidsequenz, die zu einer Nucleotidsequenz eines der Nucleinsäuremoleküle von a) bis c) komplementär ist.

Die vorliegende Erfindung löst das ihr zugrundeliegende technische Problem durch die Bereitstellung von Verwendungsmöglichkeiten von Nucleinsäuren, die hyphenspezifische Zellwandproteine von *Candida* codiert. Die erstmals bereitgestellten hyphenspezifischen Zellwandproteine Rbr1p, Rbr2p und Rbr3p sind pH- und/oder Temperatur-regulierte Virulenzfaktoren von *C*. *albicans.* Rbr1p, Rbr2p und Rbr3p sind notwendig für eine erfolgreiche Infektion des Wirtes durch Candida. Dies wurde durch die Erfinder der vorliegenden Erfindung insbesondere am Beispiel von Rbr1 p in einem Maus-Modell für systemische Candidose nachgewiesen. Untersuchungen der Erfinder der Erfindung zeigen, dass Rbr1p, Rbr2p und Rbr3p in Abhängigkeit vom pH-Wert in der Zelle exprimiert werden. Insbesondere für Rbr1p wurde gezeigt, dass es sich um ein GPI-verankertes Zellwandprotein handelt. Die hyphenspezifischen Zellwandproteine Rbr1p, Rbr2p und Rbr3p sind als Bestandteile der Zellwand besonders gut für die Entwicklung von Antimykotika geeignet, da Zellwandproteine insbesondere für kleine Wirkstoff-Moleküle frei zugänglich sind, wobei diese keine Zellmembran überwinden müssen. Durch die Identifizierung von Rbr1p, Rbr2p und Rbr3p als essentielle Faktoren für eine systemische *Candida*-Infektion werden neue, bislang noch nicht beschriebene Targets bereitgestellt, die sich in besonderem Maße zur Antimykotika-Entwicklung eignen.

Da die Zellwand von *Candida* für die Adhäsion von *Candida*-Zellen an Zellen des Wirtsorganismus verantwortlich ist, sind die Bestandteile der Zellwand hyphaler Zellen, also auch Rbr1p, Rbr2p und Rbr3p, von besonderer Bedeutung für die Ausprägung der Pathogenität von *C*. *albicans.* Da die Proteine Rbr1p, Rbr2p und Rbr3p nur in der Zellwand der virulenten Hyphenform vorkommen, lassen sich diese Zellwandproteine erfindungsgemäß auch als serodiagnostische Marker einsetzen, um beispielsweise eine invasive *Candida-*Mykose von einer einfachen *Candida*-Kolonisierung zu unterscheiden, wobei durch den Nachweis des erfindungsgemäßen Rbr1p-, Rbr2p- und/oder Rbr3p-Proteins in einer zu untersuchenden Probe das Vorliegen einer *Candida*-Infektion angezeigt wird.

Erfindungsgemäß lassen sich die Rbr1p-, nicht erfindungsgemäß auch Rbr2p- und Rbr3p-Proteine in vorteilhafter Weise auch zur Entwicklung diagnostischer und/oder therapeutischer Antikörper einsetzen, mit deren Hilfe eine Spezies-spezifische Diagnose oder Therapie möglich ist. Alle Bestandteile der Zellwand von *Candida*-Zellen spielen eine essentielle Rolle bei der Auslösung und Modulation der gegen *Candida* gerichteten Immunreaktionen des Wirtsorganismus. Im Allgemeinen besteht die *Candida*-Zellwand aus komplexen Glucose-Polymeren, beispielsweise β-1,3-Glucanen und β-1,6-Glucanen, Chitin und Mannoproteinen. Gegen diese Antigene gerichtete Antikörper weisen daher stets auch diese KohlenhydratReste nach. Da die Mannane der einzelnen *Candida*-Spezies allerdings hohe Ähnlichkeit aufweisen, besteht zwischen den *Candida-*Spezies Kreuzreaktivität, sodass eine Unterscheidung einzelner *Candida*-Spezies unter Verwendung von Antikörpern, die gegen Glucose-Polymere gerichtet sind, häufig nicht möglich ist. Gegen die Zellwandproteine Rbr1p, Rbr2p oder Rbr3p gerichtete Antikörper weisen jedoch nur Protein-spezifische Epitope nach, nicht jedoch unspezifische Mannan-Bestandteile.

Im Zusammenhang mit der vorliegenden Erfindung umfasst der Begriff "*Candida*" Sprosspilze der *Fungi imperfecti.* Sprosspilze von *Candida* sind pleomorphe Organismen, deren Lebenszyklus durch reversible morphogentische Übergänge zwischen Sprossung und pseudohyphalen beziehungsweise hyphalen Wachstumsformen gekennzeichnet sind. Der Übergang von getrennten Hefezellen (Blastosporen) zu filamentösen Formen, das heißt Hyphen und Pseudohyphen, wird als Dimorphismus bezeichnet. Gene beziehungsweise die von diesen Genen codierten Proteine, die während des hyphalen Wachstums exprimiert werden, sind von enormer Bedeutung für die Ausprägung der Virulenzeigenschaften von *Candida.* Der Übergang zwischen filamentösem Wachstum und Virulenzeigenschaften kommt beispielsweise dadurch zum Ausdruck, dass *C*. *albicans*-Formen, die keine Hyphen ausbilden, in tierischem Modellsystem, beispielsweise *Mus musculus,* avirulent sind (Lo et al., Cell, 90 (1997), 939-949). Zu diesen fakultativ pathogenen Pilzen gehören neben *C*. *albicans* auch *C*. *tropicalis, C*. *krusei, C*. *parapsilosis, C*. *guilliermondii, C*. *glabrata, C*. *dubliniensis, C*. *lusitaniae, Trichosporon-Arten oder Blastoschi*zomyces-Arten.

Unter "Differenzierungsstadien" oder "Wachstumsstadien" von *Candida*-Zellen werden erfindungsgemäß die im *Candida*-Lebenszyklus auftretenden morphogenetischen Wachstumsformen verstanden, wobei insbesondere zwischen dem Hefe-Wachstumsstadium und dem Hyphen- und Pseudohyphen-Wachstumsstadium unterschieden wird. Der Übergang vom Hefewachstum zum filamentösen Wachstum, das heißt Bildung von Hyphen oder Pseudohyphen, wird als Dimorphismus bezeichnet. Das nicht-virulente Hefe-Stadium ist durch das Auftreten sogenannter Blastosporen, bei denen es sich um gram-positive kapsellose Sprosszellen von ovaler bis rundlicher Form handelt, und die Vermehrung mittels Sprossung gekennzeichnet. Das virulente Hyphen- und Pseudohyphen-Wachstumsstadium ist durch die Bildung von echtem Myzel und/oder Pseudomyzel gekennzeichnet. Unter erfindungsgemäßer Verwendung der Nucleinsäuremoleküle, die ein während des Hyphenwachstums exprimiertes Zellwandprotein codieren, ist es also möglich, das Hyphenstadium von *Candida*-Zellen vom Hefestadium zu unterscheiden und daher das Hyphenstadium zu charakterisieren und/oder nachzuweisen, wobei das Hyphenstadium dadurch charakterisiert ist, dass das erfindungsgemäße Zellwandprotein Rbr1p und/oder das Zellwandprotein Rbr2p und/oder das Zellwandprotein Rbr3p vorliegt/vorliegen.

Die erfindungsgemäß verwendeten Nucleinsäuremoleküle sind also proteincodierende Nucleinsäuren, die ein zur Hyphenentwicklung eines pathogenen pilzlichen Organismus erforderliches Zellwandprotein, insbesondere erfindungsgemäß das Zellwandprotein Rbr1p, das Zellwandprotein Rbr2p und/oder das Zellwandprotein Rbr3p codieren. Insbesondere handelt es sich um Nucleinsäuremoleküle, die die Sequenz des *RBR1*-Gens, des *RBR2*-Gens und/oder des *RBR3-*Gens von *C*. *albicans* aufweisen. Die *RBR1-, RBR2-* und *RBR3-*Gene ist sind pH- und/oder Temperatur-regulierte Gene, deren Expression durch den Repressor Nrg1p aktiviert und durch den Transkriptionsfaktor Rim101 p reprimiert wird.

Offenbart sind auch Nucleinsäuremoleküle, die ein hyphenspezifisches Zellwandprotein einer *Candida*-Spezies codieren, wobei die Nucleinsäuremoleküle zu den Nucleinsäuren, die das Rbr1p-, Rbr2p- oder Rbr3p-Protein von *C*. *albicans* codieren, über ihre gesamte Länge hinweg eine sehr große Homologie aufweisen, wobei die Homologie mehr als 75 %, insbesondere mehr als 80 %, vorzugsweise mehr als 85 %, besonders bevorzugt mehr als 90 % und am bevorzugtesten mehr als 95 % oder mehr als 98 % beträgt. Im Zusammenhang mit der vorliegenden Erfindung wird unter "Homologie" der Grad der Identität verstanden, der zwischen den Nucleotidsequenzen von zwei Nucleinsäuremolekülen oder zwischen den Aminosäuresequenzen von zwei Protein-Molekülen besteht. Homologe Nucleinsäuren können mit der in SEQ ID Nr. 1, SEQ ID Nr. 3 oder SEQ ID Nr. 5 dargestellten Nucleinsäuresequenz hybridisieren. Hybridisieren bedeutet im Rahmen der Erfindung eine Hybridisierung unter vorzugsweise stringenten Hybridisierungsbedingungen, wie sie in Sambrook et al., "Molecular cloning: A laboratory manual", Cold Spring Harbor Laboratory Press, 2. Ausgabe (1989), beschrieben sind.

Das Nucleinsäuremolekül kann sowohl ein Wildtyp-Zellwandprotein, beispielsweise Rbr1p von *C*. *albicans* mit der in SEQ ID Nr. 2 dargestellten Aminosäuresequenz, Rbr2p von *C*. *albicans* mit der in SEQ ID Nr. 4 dargestellten Aminosäuresequenz oder Rbr3p von *C*. *albicans* mit der in SEQ ID Nr. 6 dargestellten Aminosäuresequenz, als auch ein mutiertes Zellwandprotein oder ein Derivat davon codieren. Nucleinsäuren, die ein gegenüber Rbr1p, Rbr2p oder Rbr3p von *C*. *albicans* mutiertes Zellwandprotein beziehungsweise ein Derivat davon codieren, weisen eine Nucleinsäuresequenz auf, die gegenüber der in SEQ ID Nr. 1, SEQ ID Nr. 3 oder SEQ ID Nr.5 dargestellten Nucleinsäuresequenz an mindestens einer, vorzugsweise mehreren Positionen geändert, das heißt mutiert ist. "Mehrere Positionen" bedeutet, dass 2 bis maximal 150 Positionen, insbesondere 2 bis maximal 120 Positionen, vorzugsweise 2 bis maximal 90 Positionen, besonders bevorzugt 2 bis maximal 60 Positionen, und am bevorzugtesten 2 bis maximal 30 Positionen, 2 bis maximal 24 Positionen, 2 bis maximal 18 Positionen, 2 bis maximal 12 Positionen oder 2 bis maximal 6 Positionen in der Nucleinsäuresequenz mutiert sind, wobei die Homologie der geänderte Positionen enthaltenden Nucleinsäuren über deren gesamte Länge hinweg zu der in SEQ ID Nr. 1, SEQ ID Nr. 3 oder SEQ ID Nr. 5 dargestellten Nucleinsäuresequenz mehr als 75 %, insbesondere mehr al 80 %, vorzugsweise mehr als 85 %, besonders bevorzugt mehr als 90 % und am bevorzugtesten mehr als 95 %, 96 %, 97 %. 98 % oder 99 % beträgt.

Die Abweichungen gegenüber der in SEQ ID Nr. 1, SEQ ID Nr. 3 oder SEQ ID Nr. 5 dargestellten Nucleinsäuresequenz, das heißt die Mutationen, können beispielsweise durch Deletionen, Substitutionen, Insertionen, Additionen, Nucleotid-Austausche und/oder Rekombination entstanden sein. Bei den Mutationen kann es sich um natürlicherweise auftretende Variationen handeln, beispielsweise um Sequenzen aus anderen, nahe verwandten Organismen oder anderen klinischen Isolaten der gleichen *Candida*-Spezies oder um auf natürliche Weise entstandene Mutationen. Es kann sich aber auch um Mutationen handeln, die mit technischen Mitteln künstlich erzeugt wurden, beispielsweise durch UV-Strahlen, Röntgenstrahlen oder chemische Agenzien. Es kann sich aber auch um Sequenzänderungen handeln, die mittels gentechnischer Verfahren erzeugt wurden. Erfindungsgemäß können die Nucleinsäuremoleküle auch solche Mutationen oder Abwandlungen der erfindungsgemäßen Nucleinsäure enthalten, die durch Fusion mit einem anderen Gen oder Bereichen eines solchen Genes aus der gleichen *Candida*-Spezies einer anderen *Candida*-Spezies oder einem anderen Organismus erzeugt wurden. Beispielsweise kann es sich um Nucleinsäuremoleküle handeln, die Bereiche des erfindungsgemäß verwendeten RBR1-Gens in Kombination mit Bereichen des RBR2-Gens und/oder Bereichen des RBR3-Gens aufweisen.

Die erfindungsgemäß verwendete Nucleinsäure beziehungsweise das erfindungsgemäß verwendete Nucleinsäuremolekül kann jedoch auch eine Nucleotidsequenz aufweisen, die gegenüber der in SEQ ID Nr. 1, und nicht erfindungsgemäß SEQ ID Nr. 3 oder SEQ ID Nr. 5 dargestellten Nucleotidsequenz verkürzt ist. Ein solches verkürztes Nucleinsäuremolekül kann ebenfalls ein vollständiges Zellwandprotein Rbr1p, Rbr2p bzw. Rbr3p oder aber nur ein Fragment davon, beispielsweise eine Protein-Domäne oder ein Epitop davon codieren. Unabhängig davon, ob das erfindungsgemäß verwendete, verkürzte Nucleinsäuremolekül ein vollständiges Zellwandprotein Rbr1p, Rbr2p bzw. Rbr3p oder nur ein Fragment davon codiert oder nicht, so muss sie in jedem Fall in der Lage sein, in Antisense-Orientierung die Protein-Expression der das Zellwandprotein Rbr1p, Rbr2p oder Rbr3p codierenden Nucleinsäure, beispielsweise einer das Wildtyp-Protein oder einer ein mutiertes Protein codierende Nucleinsäure, in deren natürlichen zellulären Umgebung oder in einem geeigneten Wirtssystem zu inhibieren. "Inhibieren" bedeutet im Kontext der vorliegenden Erfindung entweder eine vollständige Blockierung oder zumindest eine teilweise Reduzierung der Transkription und/oder Translation der zu inhibierenden Nucleinsäure.

In einer weiteren Ausführungsform handelt es sich bei dem erfindungsgemäß verwendeten Nucleinsäuremolekül um ein Molekül mit einer Nucleotidsequenz, die zu einer Nucleotidsequenz einer der vorgenannten Nucleinsäuremoleküle komplementär ist. Eine Nucleinsäure beziehungsweise eine Nucleotidsequenz ist "komplementär" zu einer anderen Nucleinsäure beziehungsweise Nucleotidsequenz, wenn sie über ihre gesamte Länge hinweg mit dieser einen Doppelstrang bilden kann, in dem alle Nucleotide nach den Regeln von Watson und Crick durch Wasserstoff-Brückenbindung gepaart vorliegen. Beispielsweise ist ein mRNA-Molekül komplementär zu einem der DNA-Stränge eines Genes, von dem es codiert wird.

Es ist insbesondere vorgesehen, dass die Nucleinsäuremoleküle in isolierter und gereinigter Form vorliegen. Erfindungsgemäß ist ferner vorgesehen, dass das Nucleinsäuremolekül als DNA-, RNA-, PNA-, LNA-Molekül oder als Mischform davon vorliegt. Bei "PNA" ("Peptide Nucleic Acid" oder "Polyamide Nucleic Acid")-Sequenzen handelt es sich um Moleküle, die nicht negativ geladen sind und in gleicher Weise wie DNA wirken (Nielsen et al., Science, 254 (1991), 1497-1500; Nielsen et al., Biochemiestry, 26 (1997), 5072-5077, Weiler et al., Nuc. Acids Res., 25 (1997), 2792-2799). PNA-Sequenzen umfassen ein Polyamid-Grundgerüst aus N-(2-Aminoethyl)-glycin-Einheiten und besitzen keine Glucose-Einheiten und keine Phosphat-Gruppen. Die unterschiedlichen Basen sind über Methylen-Carbonyl-Bindungen an das Grundgerüst gebunden. "LNA" (Locked Nucleic Acid)-Moleküle sind dadurch gekennzeichnet, dass die Furanose-Ring-Konformation durch einen Methylen-Linker beschränkt ist, der die 2'-O-Position mit der 4'-C-Position verbindet. LNAs werden als einzelne Nucleotide in Nucleinsäuren, beispielsweise DNA- oder RNA, eingebaut. LNA-Moleküle unterliegen ebenfalls wie PNA-Moleküle den Watson-Crick-Basenpaarungs-Regeln und hybridisieren mit komplementären DNA- und/oder RNA-Molekülen. LNA/DNA- oder LNA/RNA-Duplexmoleküle zeigen gegenüber ähnlichen Duplexmolekülen, die ausschließlich aus DNA oder RNA gebildet sind, erhöhte thermische Stabilität.

Die erfindungsgemäß eingesetzten Nucleinsäuremoleküle können aus einer natürlichen Quelle isoliert worden sein, vorzugsweise aus Zellen von *C*. *albicans* oder einer anderen *Candida*-Spezies. Die erfindungsgemäß eingesetzten Nucleinsäuremoleküle können aber auch mittels eines bekannten Verfahrens chemisch synthetisiert worden sein.

Eine weitere Ausführungsform der vorliegenden Erfindung betrifft die erfindungsgemäße Verwendung eines Fragments eines Nucleinsäuremoleküles, dass ein zur Hyphenentwicklung eines pathogenen pilzlichen Organismus erforderliches Zellwandprotein, insbesondere das Zellwandprotein Rbr1p, Rbr2p oder Rbr3p von *C*. *albicans,* codiert. Unter einem "Fragment" eines Nucleinsäuremoleküls wird ein Teilbereich eines erfindungsgemäßen Nucleinsäuremoleküls verstanden, das in der natürlichen zellulären Umgebung des erfindungsgemäßen Nucleinsäuremoleküls oder in einem geeigneten Wirtssystem die Protein-Expression der erfindungsgemäßen Nucleinsäure, die ein Zellwandprotein codiert, inhibieren kann. "Inhibieren" bedeutet im Kontext der vorliegenden Erfindung entweder eine vollständige Blockierung oder zumindest eine teilweise Reduzierung der Transkription und/oder Translation der zu inhibierenden Nucleinsäure. Eine in Antisense-Orientierung vorliegende Nucleinsäure oder ihre mRNA ist zu der mRNA einer zu inhibierenden Nucleinsäure komplementär und verbindet sich mit dieser zu einem doppelsträngigen Nucleinsäuremolekül, das enzymatisch abgebaut wird. In einer besonders bevorzugten Ausführungsform der Erfindung weist das Fragment mindestens 10 Nucleotide auf. In weiteren Ausführungsformen weist das Fragment mindestens 15, insbesondere mindestens 25, vorzugsweise mindestens 50 und besonders bevorzugt mindestens 100 Nucleotide auf. Das Fragment kann daher auch ein Oligonucleotid sein. Die Homologie des Fragmentes über seine gesamte Länge hinweg zu der in SEQ ID Nr. 1, SEQ ID Nr. 3 oder SEQ ID Nr. 5 dargestellten Nucleinsäuresequenz beträgt mindestens 75 %, insbesondere mindestens 80 %, vorzugsweise mindestens 85 %, besonders bevorzugt mindestens 90 %, und am bevorzugtesten mindestens 95 %, 96 %, 97 %, 98 % oder 99 %.

Weitere Ausführungsformen der Erfindung betreffen die Verwendung eines Vektors, der mindestens ein erfindungsgemäßes Nucleinsäuremolekül und/oder mindestens ein Nucleinsäure-Fragment unter der funktionellen Kontrolle von mindestens einem Expressions-Regulationselement enthält, dass die Transkription der Nucleinsäure in eine translatierbare RNA und/oder die Translation der RNA in ein Protein gewährleistet. Bei dem Vektor handelt es sich vorzugsweise um ein Plasmid, ein Cosmid, ein Virus, einen Bakteriophagen, einen Shuttle-Vektor oder einen anderen in der Gentechnik üblicherweise eingesetzten Vektor. Geeignete regulatorische Elemente sind beispielsweise Promotoren, Enhancer, Operatoren, Silencer, Ribosomen-Bindungsstellen und/oder Transkriptions-Terminationssignale, beispielsweise einen 3'-Transkriptionsterminator. Die regulatorischen Elemente, die funktionell mit den erfindungsgemäßen Nucleinsäuremolekülen, beispielsweise dem Nucleinsäuremolekül mit der in SEQ ID Nr. 1 dargestellten Nucleinsäuresequenz, verbunden sind, können aus anderen Organismen oder anderen Genen stammen als die Nucleinsäuremoleküle selbst. Selbstverständlich können die funktionell mit den erfindungsgemäßen Nucleinsäuremolekülen verbundenen Regulationselemente auch aus *Candida,* insbesondere *C*. *albicans* stammen. Erfindungsgemäß ist ebenfalls vorgesehen, dass der Vektor eine Signalsequenz zum Transport des von dem erfindungsgemäßen Nucleinsäuremolekül codierten, exprimierten Proteins in eine Zellorganelle, ein Zellkompartiment, in den extrazellulären Raum oder aus der Zelle heraus aufweist. In einer besonders bevorzugten Ausführungsform der vorliegenden Erfindung können die erfindungsgemäßen Nucleinsäuremoleküle in Antisense-Orientierung zu dem/den funktionell damit verbundenen regulatorischen Element(en) vorliegen, sodass eine Expression einer Antisense-mRNA ermöglicht wird, die in einem Zielorgan die Expression des endogenen *C. albicans-RBR1-Gens,* des endogenen *C*. *albicans-RBR2-*Gens beziehungsweise des endogenen *C*. *albicans-RBR3-Gens* inhibieren oder reduzieren kann. Bei Verwendung eines Fragmentes der erfindungsgemäßen Nucleinsäuremoleküle muss dieses eine Länge aufweisen, die ausreicht, um eine Hybridisierung und eine Translations-Inhibition zu ermöglichen, beispielsweise eine Länge von mindestens 10 Nucleotiden, insbesondere mindestens 15, mindestens 25, vorzugsweise mindestens 50 und besonders bevorzugt mindestens 100 Nucleotiden.

Die erfindungsgemäß verwendeten Vektoren können noch weitere Funktionseinheiten besitzen, die beispielsweise eine Stabilisierung und/oder Replikation des Vektors in einem Wirtsorganismus bewirken oder zumindest dazu beitragen. Die erfindungsgemäßen Vektoren können selbstverständlich auch Elemente wie Antibiotikaresistenz-Gene, Selektionsmarker oder Nucleinsäure-Bereiche enthalten, die Affinitätsepitope wie HA, Myc, Maltose-binding-Protein, His6 oder fluoreszierende Proteine wie Luciferase oder Gfp codieren.

Die vorliegende Erfindung betrifft insbesondere eine Wirtszelle, die eine oder mehrere der erfindungsgemäßen Nucleinsäuremoleküle und einen oder mehrere der vorstehend genannten Vektoren enthält und in bevorzugter Ausführungsform in der Lage ist, das erfndungsgemäße Zellwandprotein Rbr1p, und nicht zur Erfindung gehörig Rbr2p und/oder Rbr3p zu exprimieren. Die vorliegende Erfindung betrifft ebenfalls auch solche Zellen, die von einer Wirtszelle abstammen, die mit den vorstehend genannten erfindungsgemäßen Nucleinsäuren und Vektoren transformiert wurde. Offenbart sind die als Wirtszellen prokaryotische oder eukaryotische Zellen wie Bakterien-, Hefe-, Pflanzen-, Insekten- oder Säugerzellen, insbesondere auch menschliche Zellen. Eine Wirtszelle kann dadurch gekennzeichnet sein, dass das eingeführte Nucleinsäuremolekül beziehungsweise das in dem Vektor enthaltene Nucleinsäuremolekül heterolog in Bezug auf die transformierte Zelle ist. Das heißt, dass das Nucleinsäuremolekül, das ein Zellwandprotein von *Candida* codiert, natürlicherweise nicht in dieser Zelle vorkommt oder aber an einem anderen Ort oder in einer anderen Kopiezahl oder Orientierung im Genom der Wirtszelle lokalisiert ist als die entsprechende natürlicherweise vorkommende Nucleinsäure. Das eingeführte erfindungsgemäße Nucleinsäuremolekül kann in Bezug auf die transformierte Zelle aber auch homolog sein.

In einer Ausführungsform ist die Wirtszelle eine gram-negative prokaryotische Zelle, besonders bevorzugt eine Enterobakterien-Zelle, insbesondere eine *Escherichia coli*-Zelle. In einer weiteren Ausführungsform kann die erfindungsgemäße Wirtszelle auch eine eukaryotische Zelle sein, insbesondere ein Pilzzelle, zum Beispiel eine Zelle aus der Gattung *Candida,* erfindungsgemäß *C*. *albicans,* eine Saccharomyces-Zelle oder eine tierische Zelle, insbesondere eine Insekten- oder Säugerzelle. Besonders bevorzugte Beispiele für geeignete *C*. *albicans*-Wirtszellen sind Abkömmlinge des Stammes SC5314 (Fonzi und Irwin, Genetics, 134 (1993), 717-728). Erfindungsgemäß ist auch vorgesehen, dass die erfindungsgemäße Wirtszelle in Form einer Zellkultur vorliegen kann.

Das der vorliegenden Erfindung zugrundeliegende technische Problem wird auch durch die Bereitstellung eines Verfahrens zur Herstellung eines zur Hyphenentwicklung eines pathogenen pilzlichen Organismus erforderlichen Zellwandproteins, erfindungsgemäß des Rbr1p-Proteins, nicht erfindungsgemäß des Rbr2p-Proteins und des Rbr3p-Proteins von *Candida albicans,* gelöst, umfassend die Kultivierung einer erfindungsgemäßen Wirtszelle in einem geeigneten Kulturmedium unter Bedingungen, die eine Expression des Zellwandproteins erlauben, und die Gewinnung des exprimierten Zellwandproteins aus der Zelle oder aus dem Medium. Dem Fachmann sind zahlreiche Verfahren zur Kultivierung von prokaryotischen und eukaryotischen Wirtszellen in Kulturmedium sowie die Kultivierungsbedingungen, die zur Expression von Proteinen führen, bekannt. Der Fachmann kennt ebenfalls zahlreiche Verfahren zur Isolierung von exprimierten Proteinen aus einer Zelle und/oder aus dem Kulturmedium.

Offenbart ist ebenfalls ein Protein, das die in SEQ ID Nr. 2 dargestellte Aminosäuresequenz aufweist und von der in SEQ ID Nr. 1 dargestellten Nucleinsäuresequenz codiert wird. In bevorzugter Ausführungsform handelt es sich bei dem Protein um das Rbr1p-Protein von *C*. *albicans,* wobei das Protein unter Verwendung des erfindungsgemäßen Verfahrens zur Herstellung des zur Hyphenentwicklung erforderlichen Zellwandproteines hergestellt werden kann.

Offenbart ist auch ein Protein, das die in SEQ ID Nr. 4 dargestellte Aminosäuresequenz aufweist und von der in SEQ ID Nr. 3 dargestellten Nucleinsäuresequenz codiert wird. In bevorzugter Ausführungsform handelt es sich bei dem Protein um das Rbr2p-Protein von *C*. *albicans,* wobei das Protein unter Verwendung des Verfahrens zur Herstellung des zur Hyphenentwicklung erforderlichen Zellwandproteines hergestellt werden kann.

Offenbart ist ferner ein Protein, das mindestens die in SEQ ID Nr. 6 dargestellte Aminosäuresequenz aufweist und von der in SEQ ID Nr. 5 dargestellten Nucleinsäuresequenz codiert wird. In bevorzugter Ausführungsform handelt es sich bei dem Protein um das Rbr3p-Protein von *C*. *albicans,* wobei das Protein unter Verwendung des Verfahrens zur Herstellung des zur Hyphenentwicklung erforderlichen Zellwandproteines hergestellt werden kann.

Die vorliegende Erfindung betrifft ebenfalls Antikörper, die spezifisch ein erfindungsgemäßes Zellwandprotein, insbesondere das erfindungsgemäße Zellwandprotein Rbr1p, und nicht erfindungsgemäß Rbr2p oder Rbr3p von *C*. *albicans,* erkennen und daran binden. Im Zusammenhang mit der vorliegenden Erfindung wird unter einem "Antikörper" ein Polypeptid verstanden, das im Wesentlichen von einem Immunglobulin-Gen oder mehreren Immunglobulin-Genen codiert wird, oder Fragmente davon, das/die ein Antigen spezifisch bindet/binden und erkennt/erkennen. Bekannte Immunglobulin-Gene umfassen sowohl die kappa-, lambda-, alpha-, gamma-, delta-, epsilon- und mu-Gene für den konstanten Bereich als auch die zahlreichen Gene für den variablen Immunglobulin-Bereich. Antikörper können beispielsweise als intakte Immunglobuline oder als eine Reihe gut charakterisierter Fragmente davon, die mittels Spaltung mit verschiedenen Peptidasen erzeugt werden, vorliegen. Der in der vorliegenden Beschreibung verwendete Begriff "Antikörper" umfasst daher sowohl intakte Antikörper-Moleküle als auch Fragmente davon. Bei den Antikörper-Fragmenten handelt es sich insbesondere um Fragmente wie Fab, F(ab')₂ und Fv, die die Epitop-Determinante binden können. Die Fragmente können entweder mittels Modifikation vollständiger, ganzer Antikörper oder mittels De novo-Synthese unter Verwendung von DNA-Rekombinationstechniken erzeugt worden sein. Der Begriff "Antikörper" umfasst auch modifizierte Antikörper, beispielsweise oligomere, reduzierte, oxidierte und/oder markierte Antikörper. Bei dem Antikörper kann es sich sowohl um einen monoclonalen als auch polyclonalen Antikörper handeln. Verfahren zur Herstellung von monoclonalen und polyclonalen Antikörpern sind im Stand der Technik bekannt.

Ein gegen das Rbr1p- (erfindungsgemäß), Rbr2p- oder Rbr3p- (nicht erfindungsgemäß) Zellwandprotein gerichteter Antikörper erkennt daher spezifisch das Rbr1p-, Rbr2p- oder Rbr'3p-Protein und bindet spezifisch daran. Vorzugsweise binden die erfindungsgemäßen Antikörper oder Antikörper-Fragmente keine anderen Antigene, beispielsweise andere an der Zellwand von *Candida* vorhandene Proteine. In bevorzugter Ausführungsform ist ein gegen das Zellwandprotein Rbr1p- (erfindungsgemäß), Rbr2p- oder Rbr3- (nicht erfindungsgemäß) Protein gerichteter Antikörper in der Lage, spezifisch nur das Zellwandprotein Rbr1p-Protein oder das Rbr2p-Zellwandprotein oder das Rbr3p-Zellwandprotein einer *Candida*-Spezies zu erkennen und daran zu binden. Das heißt, ein gegen das Rbr1 p-, Rbr2p- oder Rbr3p-Protein von *C*. *albicans* gerichteter Antikörper erkennt und bindet ausschließlich das Rbr1 p-, Rbr2p- oder Rbr3p-Protein von *C*. *albicans,* nicht jedoch das entsprechende Rbr1pProtein, das entsprechende Rbr2p-Protein beziehungsweise das entsprechender Rbr3p-Protein einer verwandten *Candida*-Spezies, beispielsweise das Rbr1p-Protein, Rbr2p- oder Rbr3p-Protein von *C*. *glabrata.* Umgekehrt erkennt und bindet ein gegen das Rbr1p-, Rbr2p- oder Rbr3p-Protein von *C*. *glabrata* gerichtete Antikörper nicht das entsprechende Protein von *C*. *albicans.*

Selbstverständlich können die erfindungsgemäßen Antikörper oder Fragmente modifiziert sein, zum Beispiel mit anderen Molekülen oder anderen Teilen davon konjugiert, assoziiert oder kovalent oder nicht-kovalent gebunden sein, zum Beispiel mit einer Farbmarkierung, einer radioaktiven Markierung, einem eine messbare Reaktion auslösenden Enzym, wie einer Phosphatase oder Peroxidase, Enzymsubstraten, fluoreszierenden Substanzen, chemilumineszenten Substanzen, cytotoxischen Agenzien, Spacem, Trägerstoffen oder ähnlichem. Die markierten, konjugierten oder nicht-modifizierten Antikörper können in löslicher oder immobilisierter Form, beispielsweise auf Trägermatrices oder Beads wie Nanopartikeln vorliegen.

Offenbart ist selbstverständlich auch einen Antikörper, der einen gegen das Zellwandprotein Rbr1p gerichteten Antikörper und/oder einen gegen das Zellwandprotein Rbr2p gerichteten Antikörper und/oder einen gegen das Zellwandprotein Rbr3p gerichteten Antikörper spezifisch erkennt und daran bindet.

Die vorliegende Erfindung betrifft auch ein Verfahren zur Charakterisierung und/oder zum Nachweis des Hyphenstadiums von *Candida-*Zellen oder Zellen pathogener pilzlicher Organismen, die mit *Candida* verwandt sind, umfassend die Inkubation der Zellen oder Zellfraktionen davon mit einem Mittel zur Identifizierung des Zellwandproteins Rbr1p (erfindungsgemäß), Rbr2p, Rbr3p (nicht efindungsgemäß), eines homologen Proteins davon und/oder eines Fragmentes davon, wobei der Nachweis des Proteins oder eines Fragmentes davon das Vorliegen des virulenten Hyphenstadiums der Zellen anzeigt.

Offenbart sind also auch Protein-Marker, nämlich die Zellwandproteine Rbr1p, Rbr2p und Rbr3p von *Candida,* mit deren Hilfe es möglich ist, das Hyphenstadium von *Candida*-Zellen eindeutig und exakt zu bestimmen. Da Rbr1p, Rbr2p und Rbr3p nur in der Zellwand von hyphal wachsenden *Candida*-Zellen vorkommen, stellen die drei Proteine ideale Marker dar, mit deren Hilfe das hyphale Differenzierungsstadium von *Candida*-Zellen, beispielsweise *C*. *albicans*-Zellen, eindeutig und schnell nachgewiesen und so zwischen Hefezellen und Hyphenzellen klar unterschieden werden kann. Die Analyse und Bestimmung des hyphalen Entwicklungsstadiums von *Candida* unter Verwendung der erfindungsgemäßen Rbr1p-, Rbr2p- und Rbr3p-Proteine liefert somit auch wichtige Informationen zur Virulenz des Pilzes.

Im Zusammenhang mit der vorliegenden Erfindung wird unter dem Begriff "immunologisches Mittel" insbesondere ein gegen das Zellwandprotein Rbr1p, Rbr2p oder Rbr3p gerichtetes Antiserum, ein gegen das Zellwandprotein Rbr1p, Rbr2p oder Rbr3p gerichteter Antikörper oder ein Fragment davon oder ein Komplex davon verstanden. Unter einem "Antiserum" wird ein Serum verstand, das Antikörper gegen ein monospezifisches Antigen oder mehrere Antigene beziehungsweise Epitope enthält. Das Antiserum wird aus spezifisch zu diesem Zweck immunisierten Tieren oder von Menschen, die eine bestimmte Erkrankung, insbesondere eine durch *Candida*-Arten verursachte Erkrankung durchgemacht haben, gewonnen. Aufgrund der Heterogenität der Immunantwort enthält das gegen ein Antigen, beispielsweise das gegen Rbr1p, Rbr2p oder Rbr3p gerichtete Antiserum unterschiedliche Antikörper, sogenannte polyclonale Antikörper, die von verschiedenen Plasma-Zellklonen produziert werden. Erfindungsgemäß ist vorgesehen, dass der Nachweis von Rbr1p, Rbr2p oder Rbr3p oder eines Fragmentes davon durch das immunologische Mittel mittels eines Fluoreszenz-Verfahrens, eines Chemieluminiszenz-Verfahrens, eines immunologischen Verfahrens oder eines radiometrischen Verfahrens erfolgt. Das Verfahren zum Nachweis des Vorliegens des Zellwandproteins Rbr1p, Rbr2p oder Rbr3p kann gegebenenfalls in Kombination mit einem mikroskopischen Verfahren und einem Bildauswertungsverfahren erfolgen.

In einer bevorzugten Ausführungsform des erfindungsgemäßen Verfahrens zur Charakterisierung und/oder zum Nachweis des Hyphenstadiums von *Candida*-Zellen ist das Zellwandprotein Rbr1p das Zellwandprotein Rbr1p von *C*. *albicans,* das von einer Nucleinsäure mit der in SEQ ID Nr. 1 dargestellten Nucleinsäuresequenz codiert wird und die in SEQ ID Nr. 2 dargestellte Aminosäuresequenz aufweist. In einer nicht erfindungsgemäßen Ausführungsform des Verfahrens zur Charakterisierung und/oder zum Nachweis des Hyphenstadiums von *Candida*-Zellen ist das Zellwandprotein Rbr2p das Zellwandprotein Rbr2p von *C*. *albicans,* das von einer Nucleinsäure mit der in SEQ ID Nr. 3 dargestellten Nucleinsäuresequenz codiert wird und die in SEQ ID Nr. 4 dargestellte Aminosäuresequenz aufweist. In noch einer weiteren nicht erfindungsgemäßen Ausführungsform des Verfahrens zur Charakterisierung und/oder zum Nachweis des Hyphenstadiums von *Candida*-Zellen ist das Zellwandprotein Rbr3p das Zellwandprotein Rbr3p von *C*. *albicans,* das von einer Nucleinsäure mit der in SEQ ID Nr. 5 dargestellten Nucleinsäuresequenz codiert wird und die in SEQ ID Nr. 6 dargestellte Aminosäuresequenz aufweist.

In weiteren Ausführungsformen des erfindungsgemäßen Verfahrens ist das Zellwandprotein Rbr1 (erfindungsgemäß), Rbr2p oder Rbr3p (nicht erfindungsgemäß) das Rbr1-Protein, Rbr2p-Protein oder Rbr3p-Protein beziehungsweise ein homologes Protein dazu von *C*. *tropicalis, C. krusei, C. parapsilosis, C. guilliermondii, C. glabrata, C. dubliniensis, C. lusitaniae,* einer *Trichosporon-Art* oder einer *Blastoschizomyces-Art,* wobei der Nachweis des Proteins vorzugsweise unter Verwendung eines Antikörpers erfolgt, der spezifisch gegen das Rbr1p-Zellwandprotein, das Rbr2p-Zellwandprotein beziehungsweise das Rbr3p-Zellwandprotein von *C*. *tropicalis, C. krusei, C. parapsilosis, C. guilliermondi, C. glabrata, C. dubliniensis, C. lusitaniae, Trichosporon* oder *Blastoschizomyces* gerichtet ist. Dadurch wird erfindungsgemäß das hyphale Differenzierungsstadium von *C*. *tropicalis, C. krusei, C. parapsilosis, C. guilliermondi, C. glabrata, C. dubliniensis, C. lusitaniae, Trichosporon* beziehungsweise *Blastoschizomyces* charakterisiert und/oder nachgewiesen. Antikörper gegen das Rbr1p-Zellwandprotein, das Rbr2p-Zellwandprotein beziehungsweise das Rbr3p-Zellwandprotein aus einer der genannten *Candida*-Spezies können beispielsweise hergestellt werden, indem das Rbr1p-Zellwandprotein, das Rbr2p-Zellwandprotein oder das Rbr3p-Zellwandprotein aus Zellen der entsprechenden *Candida-*Spezies isoliert und dann unter Verwendung von dem Fachmann bekannten Verfahren zur Erzeugung eines monoclonalen oder polyclonalen Antikörpers eingesetzt wird. Das Rbr1p-Zellwandprotein, das Rbr2p-Zellwandprotein beziehungsweise das Rbr3p-Zellwandprotein einer *Candida*-Spezies kann auch unter Verwendung einer der in SEQ ID Nr. 1, SEQ ID Nr. 3 oder SEQ ID Nr. 5 dargestellten Nucleinsäuresequenzen erhalten werden, indem diese zur Isolierung einer homologen Nucleinsäure aus Zellen der entsprechenden *C*an*dida*-Spezies eingesetzt wird. Nach Isolierung und Sequenzierung der homologen Nucleinsäure kann dann auf der Basis der erhaltenen Nucleinsäuresequenz die Aminosäuresequenz des von der Nucleinsäure codierten Rbr1p-Zellwandproteines, die Aminosäuresequenz des von der Nucleinsäure codierten Rbr2p-Zellwandproteines beziehungsweise die Aminosäuresequenz des von der Nucleinsäure codierten Rbr3p-Zellwandproteines abgeleitet werden. Das entsprechende Protein kann dann beispielsweise in vitro chemisch synthetisiert und zur Herstellung eines spezifisch dagegen gerichteten Antikörpers eingesetzt werden.

Erfindungsgemäß ist vorgesehen, dass die zu charakterisierenden *Candida*-Zellen in einer Probe, vorzugsweise einer biologischen Probe vorliegen. Im Zusammenhang mit der vorliegenden Erfindung wird unter einer "biologischen Probe" insbesondere ein Haut- oder Schleimhautabstrich, eine Organbiopsie, eine Gewebebiopsie, eine Körperflüssigkeit, ein Körpersekret, Stuhl oder eine Spülflüssigkeit verstanden, die durch Spülung von Hohlräumen oder Hohlorganen eines menschlichen oder tierischen Körpers gewonnen wurde. Die Probe kann sowohl einem lebenden oder toten Organismus, Organ oder Gewebe entnommen worden sein. Bei einer "Biopsie" handelt es sich um eine durch Punktion mit einer Hohlnadel, unter Anwendung eines speziellen Instrumentes wie eines Stanzinstrumentes, einer Biopsiesonde oder einer Zange oder operativ mit dem Skalpell gewonnene Gewebeprobe. Bei einer "Körperflüssigkeit" handelt es sich insbesondere um Sputum, Urin, Pleuraerguss, Liquor, Lymphe oder Blut. Erfindungsgemäß bevorzugt kann das Blut sowohl eine ungereinigte als auch eine gereinigte Blutprobe, Blutplasma oder Blutserum sein. "Sekrete" sind Absonderungen von Zellen, insbesondere Drüsen.

Eine "biologische Probe" kann erfindungsgemäß auch eine Kultur, beispielsweise eine Blutkultur, also ein Keimanzüchtung aus einer Blutprobe, oder ein Kulturmedium sein, beispielsweise ein Fermentationsmedium, in dem *Candida*-Zellen oder menschliche, tierische oder pflanzliche Zellen kultiviert wurden. Bei einer Probe im Sinne der Erfindung kann es sich auch um eine wässrige Lösung, Emulsion, Dispersion oder Suspension handelt, die isolierte und aufgereinigte *Candida*-Zellen oder Bestandteile davon enthält. Eine biologische Probe kann bereits Aufreinigungsschritten unterworfen worden sein, um *Candida*-Zellen zu isolieren oder anzureichern, kann aber auch ungereinigt vorliegen.

In einer bevorzugten Ausführungsform des erfindungsgemäßen Verfahrens handelt es sich bei den eingesetzten, zu charakterisierenden *Candida*-Zellen um Zellen, die aus einer biologischen Probe isoliert und angereichert wurden und als Zellpräparat, vorzugsweise in einem geeigneten Puffersystem vorliegen. Bei den *Candida*-Zellen handelt es sich vorzugsweise um intakte Zellen.

Erfindungsgemäß ist ebenfalls vorgesehen, dass zur Charakterisierung und/oder zum Nachweis des hyphalen Differenzierungsstadiums von *Candida*-Zellen auch Zellfraktionen eingesetzt werden, die durch Aufschluss und Fraktionierung von *Candida*-Zellen erhalten werden, wobei isolierte und vorzugsweise aufgereinigte Zellfraktionen erhalten werden. Diese so erhaltenen Zellfraktionen können dann im erfindungsgemäßen Verfahren zur Charakterisierung und/oder zum Nachweis des Hyphenwachstumsstadiums von *Candida*-Zellen eingesetzt werden. Verfahren zum Zellaufschluss und zur Fraktionierung von Zellbestandteilen sind auf dem Fachgebiet gut bekannt. Vorzugsweise umfassen die zur Charakterisierung der Differenzierungsstadien von *Candida*-Zellen eingesetzten Zellfraktionen mindestens eine Zellwand-Fraktion der *Candida*-Zellen.

Das der vorliegenden Erfindung zugrundeliegende technische Problem wird auch durch ein Verfahren zum Nachweis einer *Candida-*Infektion in einer aus einem menschlichen oder tierischen Organismus erhaltenen biologischen Probe gelöst, wobei die Gegenwart des Proteins Rbr1p (erfindungsgemäß), Rbr2p (nicht erfindungsgemäß), Rbr3p (nicht erfindungsgemäß), eines homologen Proteins davon und/oder eines Fragmentes davon in der biologischen Probe und/oder in der Zellwand von gegebenenfalls in der biologischen Probe enthaltenen *Candida*-Zellen oder Zellen von mit *Candida* verwandten pathogenen Organismen nachgewiesen wird, umfassend
a) die Inkubation der biologischen Probe mit einem Mittel zur Identifizierung des Proteins Rbr1p, Rbr2p, Rbr3p, eines homologen Proteins davon und/oder eines Fragmentes davon und
b) den Nachweis der Interaktion des Identifizierungsmittels mit dem Protein oder Fragment davon.

Das Verfahren zum Nachweis einer *Candida*-Infektion beruht also entweder auf dem direkten Nachweis des in der Probe frei vorliegenden Rbr1p- (erfindungsgemäß), Rbr2p- oder Rbr3p- (nicht erfindungsgemäß) Zellwandproteins von *Candida* beziehungsweise eines Teils davon oder aber auf dem Nachweis, dass das Zellwandprotein Rbr1p, Rbr2p oder Rbr3p in der Zellwand von *Candida*-Zellen, die in der Probe enthalten sind, vorhanden ist. In beiden Fällen weist der Nachweis von Rbr1p, Rbr2p und/oder Rbr3p auf das Vorkommen virulenter hyphaler *Candida*-Zellen und damit auf das Vorliegen einer *Candida*-Infektion hin. Das erfindungsgemäße Verfahren erlaubt somit in vorteilhafter Weise einen schnellen und exakten Nachweis einer *Candida*-Infektion, insbesondere einer invasiven Candidiasis. Das erfindungsgemäße Verfahren ermöglich also insbesondere eine sehr frühe Diagnose der Mykose.

Der erfindungsgemäße direkte Nachweis des *Candida-*Zellwandproteins Rbr1p, und nicht erfindungsgemäß Rbr2p und/oder Rbr3p in einer Probe oder in der Zellwand von in der Probe enthaltenen *Candida*-Zellen, insbesondere der immunologische Nachweis des Zellwandantigens mittels eines gegen das Zellwandprotein Rbr1p, das Zellwandprotein Rbr2p oder das Zellwandprotein Rbr3p gerichteten Antikörpers, hat gegenüber den derzeit bekannten Diagnose-Verfahren einen erheblich höheren diagnostischen Wert. So ermöglicht der derzeit durchgeführte kulturelle und/oder histologische Nachweis von *Candida* in den meisten Fällen lediglich den Nachweis einer *Candida*-Infektion im Spätstadium, während sich frühe Stadien insbesondere bei immunsupprimierten Patienten diesem Nachweis häufig entziehen. Auch die derzeit erfassten Antikörper-Titer gegen *Candida*-Antigene eignen sich bislang kaum oder überhaupt nicht zur Diagnose einer *Candida*-Infektion. Die Ursachen dafür sind vielfältig. So ist bekannt, dass gesunde Personen Antikörper gegen *Candida*-Antigene aufweisen. Andererseits entwickeln manche Patienten, insbesondere immunsupprimierte Patienten, bei einer *Candida*-Infektion sehr häufig keine adäquate Immunreaktion. Eine weitere Ursache besteht darin, dass häufig Serumproben entnommen werden, bevor sich Antikörper gebildet haben.

Im Zusammenhang mit der vorliegenden Erfindung wird unter einer "*Candida*-Infektion" eine "*Candida*-Mykose" oder Candidose oder Candidiasis verstanden, also eine Infektion, Krankheit oder ein Krankheitszustand, der durch eine *Candida*-Art verursacht wird. Insbesondere handelt es sich um solche Erkrankungen, die ausschließlich von *Candida*-Arten verursacht werden. Der Begriff "*Candida-*Infektion" umfasst alle Erkrankungen, die von *Candida*-Arten wie *C*. *albicans, C*. *tropicalis, C*. *krusei, C*. *parapsilosis, C*. *guilliermondi, C*. *glabrata, C*. *dubliniensis, C*. *lusitaniae,* einer *Trichosporon-Art* oder einer *Blastoschizomyces-Art* verursacht werden. Im Kontext mit der Erfindung werden unter dem Begriff "*Candida*-Infektionen" auch Krankheiten oder Krankheitszustände verstanden, die primär andere Ursachen haben und bei denen die vorstehend genannten *Candida-*Arten am Gesamtkrankheitsbild nur beteiligt sind beziehungsweise zusätzliche Symptome hinzufügen, zum Beispiel opportunistische Infektionen.

Unter einer invasiven "Candidiatis" werden erfindungsgemäß *C*andi*da*-Infektionen im Blutkreislauf und in inneren Organen verstanden. Invasive Candidiatis tritt insbesondere dann auf, wenn Zellen einer *Candida*-Spezies in den Blutkreislauf gelangen, dort zu einer Infektion führen und sich von da aus über den gesamten Körper verteilen. Zu den häufigsten Symptomen invasiver Candidiatis gehören Fieber und Schüttelfrost. Wenn sich die Infektion zu tiefer gelegenen Organen wie Nieren, Leber oder Augen ausbreitet, können sich zusätzliche spezifische Symptome entwickeln.

Erfindungsgemäß ist vorgesehen, dass zur Identifizierung von Rbr1p, und nicht erfindungsgemäß Rbr2p oder Rbr3p als Zelloberflächen-Antigen von *Candida*-Zellen oder aber als freies, insbesondere in Körperflüssigkeiten zirkulierendes Antigen ein immunologisches Mittel eingesetzt wird, insbesondere ein gegen Rbr1p, Rbr2p oder Rbr3p gerichtetes Antiserum, ein gegen Rbr1p, Rbr2p oder Rbr3p gerichteter Antikörper oder ein Fragment davon oder ein Komplex davon. Der erfindungsgemäß eingesetzte Antikörper kann sowohl ein monoclonaler als auch ein polyclonaler Antikörper sein, der vorzugsweise eine Markierung, insbesondere eine Farbmarkierung, eine radioaktive Markierung, eine Fluoreszenzmarkierung, eine Chemielumineszenz-Markierung oder ein eine messbare Reaktion auslösendes Enzym aufweist. Die Untersuchung der Interaktion des Identifizierungsmittels, vorzugsweise des Antikörpers, mit dem Rbr1p-Zellwandantigen (erfindungsgemäß), Rbr2p-Zellwandantigen oder Rbr3p-Zellwandantigen (nicht erfindungsgemäß) erfolgt mittels eines immunologischen Verfahrens, eines Fluoreszenz-Verfahrens, eines Chemielumineszenz-Verfahrens oder eines radiometrischen Verfahrens, gegebenenfalls in Kombination mit einem mikroskopischen Verfahren und/oder einem Bildauswertungsverfahren.

In bevorzugter Ausführungsform ist das nachzuweisende Protein das Zellwandprotein Rbr1p von *C*. *albicans,* das von einem Nucleinsäuremolekül mit der in SEQ ID Nr. 1 dargestellten Nucleinsäuresequenz codiert wird und das die in SEQ ID Nr. 2 dargestellte Aminosäuresequenz aufweist. In einer weiteren nicht erfindungsgemäßen Ausführungsform ist das nachzuweisende Protein das Zellwandprotein Rbr2p von *C. albicans,* das von einem Nucleinsäuremolekül mit der in SEQ ID Nr. 3 dargestellten Nucleinsäuresequenz codiert wird und das die in SEQ ID Nr. 4 dargestellte Aminosäuresequenz aufweist. In noch einer weiteren nicht erfindungsgemäßen Ausführungsform ist das nachzuweisende Protein das Zellwandprotein Rbr3p von *Candida albicans,* das von einem Nucleinsäuremolekül mit der in SEQ ID Nr. 5 dargestellten Nucleinsäuresequenz codiert wird und das die in SEQ ID Nr. 6 dargestellte Aminosäuresequenz aufweist. Durch den Nachweis von Rbr1p (erfindungsgemäß), Rbr2p und/oder Rbr3p von *C*. *albicans* in der Probe beziehungsweise den Nachweis von *C*. *albicans*-Rbr1p (erfindungsgemäß), *C*. *albicans*-Rbr2p und/oder *C*. *albicans*-Rbr3p in der Zellwand von *Candida*-Zellen, die in der biologischen Probe enthalten sind, können invasive Pilzerkrankungen nachgewiesen werden, die insbesondere durch *C*. *albi*cans verursacht werden, insbesondere eine durch *C*. *albicans* verursachte invasive Candidiadis. Durch den Nachweis des Rbr1p-Proteins, (erfindungsgemäß) Rbr2p-Proteins oder Rbr3p-Proteins von *C. tropicalis, C. krusei, C. parapsilosis, C. guilliermondii, C. glabrata, C. dubliniensis, C. lusitaniae* einer Trichosporon-Art oder einer Blastoschizomyces-Art können invasive Pilzerkrankungen nachgewiesen werden, die insbesondere von *C. tropicalis, C. krusei, C. parapsilosis, C. guilliermondii, C. glabrata, C. dubliniensis, C. lusitaniae,* einer Trichosporon-Art beziehungsweise einer Blastoschizomyces-Art verursacht werden, insbesondere eine durch eine der genannten *Candida*-Spezies verursachte invasive Candidiadis.

Erfindungsgemäß ist ferner vorgesehen, dass der Nachweis der *Candida*-Infektion unter Verwendung einer biologischen Probe erfolgt, die *Candida*-Zellen enthalten kann, aber nicht enthalten muss. Die biologische Probe kann beispielsweise ein Haut- oder Schleimhautabstrich, eine Organbiopsie, eine Gewebebiopsie, eine Körperflüssigkeit, ein Körpersekret, Stuhl oder eine Spülflüssigkeit sein.

In einer bevorzugten Ausführungsform der Erfindung erfolgt der Nachweis der *Candida*-Infektion unter Verwendung einer Körperflüssigkeit, die im Vergleich zu anderen Proben wie einer Biopsie den Vorteil aufweisen, dass die Wahrscheinlichkeit sehr hoch ist, dass Rbr1p, Rbr2p und/oder Rbr3p frei zirkulieren. Bei der Körperflüssigkeit handelt es sich insbesondere um eine Blutprobe, die dem Körper direkt entnommen und ohne weitere Aufreinigung im efindungsgemäßen Verfahren eingesetzt werden kann. Das erfindungsgemäße Verfahren kann auch unter Verwendung einer Blutkultur durchgeführt werden. Ebenfalls können erfindungsgemäß Blutprodukte wie Blutplasma oder Blutserum eingesetzt werden. Der Nachweis des frei im Blut zirkulierenden Zellwandproteins Rbr1p, Rbr2p und/oder Rbr3p beziehungsweise den Nachweis, dass im Blut enthaltene *Candida*-Zellen auf ihrer Zelloberfläche Rbr1p, Rbr2p und/oder Rbr3p aufweisen, kann eine invasise Candidiadis im Blut nachgewiesen werden. Dass zur Identifizierung von Rbr1p, Rbr2p und/oder Rbr3p eingesetzte Mittel ist vorzugsweise ein immunologisches Mittel, insbesondere ein gegen Rbr1p, Rbr2p oder Rbr3p gerichtetes Antiserum, ein gegen Rbr1p, Rbr2p oder Rbr3p gerichteter Antikörper oder ein Fragment davon oder ein Komplex davon. Im Falle der Verwendung eines Antikörpers kann dieser eine Markierung, ausgewählt aus der Gruppe bestehend aus einer Farbmarkierung, einer radioaktiven Markierung, einer Fluoreszenzmarkierung, einer Chemielumineszenz-Markierung oder einem eine messbare Reaktion auslösenden Enzym, aufweisen.

Die vorliegende Erfindung betrifft ebenfalls ein Verfahren zum Auffinden und Identifizieren von Substanzen mit therapeutischer Wirkung gegen Krankheiten, die durch *Candida*-Arten oder pathogene pilzliche Arten, die mit *Candida* verwandt sind, verursacht werden. Erfindungsgemäß ist dabei vorgesehen, dass eine zu testende Substanz in einem geeigneten Medium mit mindestens einem Agens, ausgewählt aus der Gruppe bestehend aus: einem Nucleinsäuremolekül, das ein zur Hyphenentwicklung eines pathogenen pilzlichen Organismus erforderliches Zellwandprotein codiert und ausgewählt ist aus der Gruppe bestehend aus:
a) einem Nucleinsäuremolekül mit einer SEQ ID Nr. 1 dargestellten Nucleotidsequenz,
b) einem Nucleinsäuremolekül mit einer Nucleotidsequenz, die ein Protein mit einer in SEQ ID Nr. 2 dargestellten Aminosäuresequenz codiert,
c) einem Nucleinsäuremolekül mit einer Nucleotidsequenz, die über ihre gesamte Länge eine Homologie von mindestens 80% zu einer Nucleotidsequenz eines der Nucleinsäuremoleküle a) oder b) zeigt,
d) einem Nucleinsäuremolekül mit einer Nucleotidsequenz, die zu einer Nucleotidsequenz eines der Nucleinsäuremoleküle von a) bis c) komplementär ist, und
e) einem Fragment eines in a) bis d) definierten Nucleinsäuremoleküls, das in einer Wirtszelle in Antisense-Orientierung zu einem Promotor die Expression eines Zellwandproteins eines pathogenen pilzlichen Organismus hemmen kann und mindestens 10 Nucleotide umfasst;
einem Vektor, der das Nucleinsäuremolekül enthält;
einer Wirtszelle, die den Vektor enthält;
einem Protein, das eine Aminosäuresequenz aus SEQ ID Nr. 2 enthält, und
einem Antikörper, der spezifisch das Protein erkennt und daran bindet
und eine Interaktion zwischen der zu testenden Substanz und dem Agens nachgewiesen wird.

Beispielsweise können die Nucleinsäuremoleküle, die Fragmente dieser Nucleinsäuremoleküle, die Proteine oder die erfindungsgemäßen Antikörper auf entsprechenden Chips immobilisiert werden. Diese Chips können dann anschließend zum Nachweis einer Interaktion zwischen einer zu testenden Substanz und dem auf den Chips immobilisierten Agens in einem geeigneten Medium mit der zu testenden Substanz in Kontakt gebracht werden. Auf diese Weise können beispielsweise die Nucleinsäuremoleküle, Proteine beziehungsweise Antikörper verwendet werden, um Substanzen, beispielsweise Proteine, zu identifizieren, die in vivo an Nucleotidsequenzen, die hyphenspezifisch exprimierte Zellwandproteine wie Rbr1p, Rbr2p oder Rbr3p codieren beziehungsweise die Expression dieser Proteine regulieren oder an die hyphenspezifisch exprimierten Zellwandproteine selbst binden. Solche bindenden Substanzen, insbesondere Proteine, können potentiell als Medikamente gegen Candida-verursachte Krankheiten eingesetzt werden, wenn sie beispielsweise in der Lage sind, durch Bindung an regulatorische Nucleotidsequenzen die Transkription der erfindungsgemäßen hyphenspezifischen Zellwandproteine zu hemmen oder zu unterbinden oder wenn sie durch Bindung an die erfindungsgemäßen hyphenspezifisch exprimierten Zellwandproteine deren Aktivität hemmen oder unterbinden können. Wenn solche Substanzen, die an Nucleinsäuremoleküle oder Proteine binden, die Transkription der erfindungsgemäß offenbarten hyphenspezifischen Zellwandproteine induzieren oder fördern oder die Aktivität der erfindungsgemäß offenbarten hyphenspezifisch exprimierten Zellwandproteine begünstigen, können diese Substanzen potenziell als Medikamente zur Behandlung *Candida*-verursachter Krankheiten verwendet werden.

Die vorliegende Erfindung betrifft ebenfalls eine diagnostische Zusammensetzung, umfassend mindestens ein

Nucleinsäuremolekül, das ein zur Hyphenentwicklung eines pathogenen pilzlichen Organismus erforderliches Zellwandprotein codiert und ausgewählt ist aus der Gruppe bestehend aus:
a) einem Nucleinsäuremolekül mit einer SEQ ID Nr. 1 dargestellten Nucleotidsequenz,
b) einem Nucleinsäuremolekül mit einer Nucleotidsequenz, die ein Protein mit einer in SEQ ID Nr. 2 dargestellten Aminosäuresequenz codiert,
c) einem Nucleinsäuremolekül mit einer Nucleotidsequenz, die über ihre gesamte Länge eine Homologie von mindestens 80% zu einer Nucleotidsequenz eines der Nucleinsäuremoleküle a) oder b) zeigt,
d) einem Nucleinsäuremolekül mit einer Nucleotidsequenz, die zu einer Nucleotidsequenz eines der Nucleinsäuremoleküle von a) bis c) komplementär ist, und
e) einem Fragment eines in a) bis d) definierten Nucleinsäuremoleküls, das in einer Wirtszelle in Antisense-Orientierung zu einem Promotor die Expression eines Zellwandproteins eines pathogenen pilzlichen Organismus hemmen kann und mindestens 10 Nucleotide umfasst;
ein Vektor, der das Nucleinsäuremolekül enthält;
eine Wirtszelle, die den Vektor enthält;
ein Protein, das eine Aminosäuresequenz aus SEQ ID Nr. 2 enthält, und/oder
ein Antikörper, der spezifisch das Protein erkennt und daran bindet.

Eine weitere bevorzugte Ausführungsform der Erfindung betrifft eine pharmazeutische Zusammensetzung, umfassend mindestens
ein Nucleinsäuremolekül, das ein zur Hyphenentwicklung eines pathogenen pilzlichen Organismus erforderliches Zellwandprotein codiert und ausgewählt ist aus der Gruppe bestehend aus:
a) einem Nucleinsäuremolekül mit einer SEQ ID Nr. 1 dargestellten Nucleotidsequenz,
b) einem Nucleinsäuremolekül mit einer Nucleotidsequenz, die ein Protein mit einer in SEQ ID Nr. 2 dargestellten Aminosäuresequenz codiert,
c) einem Nucleinsäuremolekül mit einer Nucleotidsequenz, die über ihre gesamte Länge eine Homologie von mindestens 80% zu einer Nucleotidsequenz eines der Nucleinsäuremoleküle a) oder b) zeigt,
d) einem Nucleinsäuremolekül mit einer Nucleotidsequenz, die zu einer Nucleotidsequenz eines der Nucleinsäuremoleküle von a) bis c) komplementär ist, und
e) einem Fragment eines in a) bis d) definierten Nucleinsäuremoleküls, das in einer Wirtszelle in Antisense-Orientierung zu einem Promotor die Expression eines Zellwandproteins eines pathogenen pilzlichen Organismus hemmen kann und mindestens 10 Nucleotide umfasst;
ein Vektor, der das Nucleinsäuremolekül enthält;
eine Wirtszelle, die den Vektor enthält;
ein Protein, das eine Aminosäuresequenz aus SEQ ID Nr. 2 enthält, und/oder
ein Antikörper, der spezifisch das Protein erkennt und daran bindet.

Die in der erfindungsgemäßen diagnostischen oder pharmazeutischen Zusammensetzung enthaltene Nucleinsäure kann also eine Protein-codierende Nucleinsäure sein, insbesondere eine Nucleinsäure, die das Zellwandprotein Rbr1p von *C*. *albicans,* und nicht erfindungsgemäß das Zellwandprotein Rbr2p von *C*. *albicans* oder das Zellwandprotein Rbr3p von *C*. *albicans* codiert. Die in der diagnostischen oder pharmazeutischen Zusammensetzung enthaltene Nucleinsäure kann aber auch eine Nucleinsäuresequenz enthalten, die Rbr1 p, und nicht erfindungsgemäß Rbr2p oder Rbr3p von *C*. *tropicalis, C. krusei, C. parapsilosis, C. guilliermondii, C. glabrata, C. dubliniensis, C. lusitaniae,* einer Trichosporon-Art oder einer Blastoschizomyces-Art codiert. Die Nucleinsäuren, die eines der Proteine aus einer der vorgenannten *Candida*-Spezies codieren, weisen zu einer *C*. *albicans*-Rbr1p, *C*. *albicans*-Rbr2p oder *C*. *albicans*-Rbr3p codierenden Nucleinsäure über ihre gesamte Länge hinweg eine sehr große Homologie auf, wobei die Homologie mehr als 75 %, insbesondere mehr als 80 %, vorzugsweise mehr als 85 %, besonders bevorzugt mehr als 90 % und am bevorzugtesten mehr als 95 % oder mehr als 98 % beträgt.

Das in der erfindungsgemäßen diagnostischen oder pharmazeutischen Zusammensetzung enthaltene Zellwandprotein Rbr1p, und nicht erfindungsgemäß Rbr2p oder Rbr3p kann in der Zusammensetzung beispielsweise als Komplex mit anderen Proteinen vorliegen. Bei diesen Proteinen handelt es sich vorzugsweise um solche, die ebenfalls zur Diagnose und/oder Therapie von *Candida-*Infektionen eingesetzt werden können.

Das in der erfindungsgemäßen diagnostischen oder pharmazeutischen Zusammensetzung enthaltene Protein kann auch ein Fragment eines Wildtyp-Proteins oder ein Derivat eines Wildtyp-Proteins sein. Unter einem "Derivat" wird ein funktionelles Äquivalent oder ein funktioneller Abkömmling des Wildtyp-Proteins verstanden, das/der unter Beibehaltung der Grundstruktur von Rbr1p, und nicht erfindungsgemäß Rbr2p oder Rbr3p durch Substitution von Atomen oder Molekülgruppen beziehungsweise Molekülresten erhalten wird und/oder dessen Aminosäuresequenz sich an mindestens einer Aminosäureposition vom Wildtyp-Protein unterscheidet.

Die Unterschiede zwischen dem Zellwandprotein-Derivat und dem Wildtyp-Zellwandprotein können auf natürlicherweise auftretenden oder künstlich erzeugten Mutationen, beispielsweise mittels auf dem Fachgebiet bekannter molekularbiologischer Techniken, in den die Derivate codierende Nucleinsäuren beruhen. Die Unterschiede können auch mittels chemischer Verfahren, beispielsweise bei der chemischen Synthese der Protein-Derivate, erzeugt worden sein. Bei Derivaten handelt es sich auch um Fusionsproteine, bei denen am N-Terminus und/oder C-Terminus von Rbr1p, Rbr2p oder Rbr3p funktionelle Domänen eines anderen Proteins enthalten sind. Die erfindungsgemäß verwendeten Derivate von Rbr1p, und nicht erfindungsgemäß Rbr2p oder Rbr3p weisen zu den entsprechenden Wildtyp-Zellwandproteinen, insbesondere zu *C*. *albicans* Rbr1p mit der in SEQ ID Nr. 2 dargestellten Aminosäuresequenz, zu *C*. *albicans* Rbr2p mit der in SEQ ID Nr. 4 dargestellten Aminosäuresequenz oder zu *C*. *albicans* Rbr3p mit der in SEQ ID Nr. 6 dargestellten Aminosäuresequenz, eine Homologie von mindestens 75 %, beispielsweise mindestens 80 %, insbesondere mindestens 85 %, vorzugsweise mindestens 90 %, bevorzugter mindestens 95 % und am bevorzugtesten mindestens 96 %, 97 %, 98 % oder 99 % auf.

Erfindungsgemäß können die in der diagnostischen oder pharmazeutischen Zusammensetzung enthaltene Protein-Derivate gegenüber dem Wildtyp-Protein eine veränderte Aktivität, eine veränderte Stabilität, eine veränderte Spezifität, ein verändertes Temperatur-, ein verändertes pH-Wert- und/oder Konzentrationsprofil und/oder ein verändertes Effektorenmuster aufweisen. Das erfindungsgemäß verwendete Zellwandprotein-Derivat kann in anderen Konformationen vorkommen und auch andere Untereinheiten beziehungsweise prä- und/oder posttranslationale Modifikationen aufweisen.

Erfindungsgemäß ist auch vorgesehen, dass die diagnostischen oder pharmazeutischen Zusammensetzungen einen Antikörper enthalten können, der spezifisch das Zellwandprotein Rbr1p (erfindungsgemäß), das Zellwandprotein Rbr2p oder das Zellwandprotein Rbr3p (nicht erfindungsgemäß) von *C*. *albicans* oder von *C*. *tropicalis, C. krusei, C. parapsilosis, C. guilliermondii, C. glabrata, C. dubliniensis, C. lusitaniae,* einer Trichosporon-Art oder einer Blastoschizomyces-Art erkennt und daran bindet und so dessen Nachweis ermöglicht. Bei dem Antikörper kann es sich sowohl um einen monoclonalen als auch einen polyclonalen Antikörper handeln. Erfindungsgemäß kann in der diagnostischen oder pharmazeutischen Zusammensetzung auch nur ein Antikörper-Fragment, beispielsweise Fab, F(ab')₂ und Fv enthalten sein.

In einer weiteren Ausführungsform kann die erfindungsgemäße diagnostische oder pharmazeutische Zusammensetzung einen Antikörper enthalten, der spezifisch einen Antikörper, der gegen Rbr1p von *C. albicans,* und nicht erfindungsgemäß gegen Rbr2p von *C*. *albicans* oder gegen Rbr3p von *C*. *albicans* gerichtet ist, erkennt und daran bindet und so den Nachweis dieses Antikörpers, beispielsweise in einer biologischen Probe wie Blut ermöglicht.

In einer besonders bevorzugten Ausführungsform handelt es sich bei der pharmazeutischen Zusammensetzung um einen Impfstoff, der mindestens ein pilzliches Zellwandprotein, insbesondere das Zellwandprotein Rbr1p von *C*. *albicans* mit der in SEQ ID Nr. 2 dargestellten Aminosäuresequenz, und nicht erfindungsgemäß das Zellwandprotein Rbr2p von *C*. *albicans* mit der in SEQ ID Nr. 4 dargestellten Aminosäuresequenz und/oder das Zellwandprotein Rbr3p von *C*. *albicans* mit der in SEQ ID Nr. 6 dargestellten Aminosäuresequenz, enthält und der zur aktiven Immunisierung eines menschlichen oder tierischen Körpers gegen eine *Candida*-Infektion geeignet ist. Im Zusammenhang mit der vorliegenden Erfindung wird unter einer "aktiven Immunisierung" eine parentarale Gabe von nicht vermehrungsfähigen Antigenen verstanden, wobei das Ziel darin besteht, im menschlichen oder tierischen Körper einen Antikörper gegen das Antigen und somit gegen den Mikroorganismus, von dem das Antigen stammt, zu bilden. Bei einer aktiven Immunisierung kann es sich auch um eine lokale Gabe, insbesondere orale, nasale oder kutane Verabreichung oder Inhalation, von nichtvermehrungsfähigen mikrobiellen Antigenen handeln, wobei das Ziel darin besteht, eine lokale Infektabwehr an Schleimhäuten durch Bildung sekretorischer Antikörper und durch Erhöhung der Makrophargenaktivität aufzubauen.

In einer weiteren bevorzugten Ausführungsform handelt es sich bei der pharmazeutischen Zusammensetzung um einen Impfstoff, der mindestens einen Antikörper ausgewählt aus der Gruppe bestehend aus erfindungsgemäß einem Antikörper, der Rbr1p von *Candida* spezifisch erkennt und daran bindet, nicht erfindungsgemäß einem Antikörper, der Rbr2p von *Candida* spezifisch erkennt und daran bindet enthält, und nicht erfindungsgemäß einem Antikörper, der Rbr3p von *Candida* spezifisch erkennt und daran bindet, enthält und der zur passiven Immunisierung eines menschlichen oder tierischen Körpers gegen eine *Candida*-Infektion geeignet ist. Im Zusammenhang mit der vorliegenden Erfindung wird unter einer "passiven Immunisierung" die Einspritzung von Immunglobulin-Präparaten, also spezifischen Antikörpern, oder Serum eines aktiv immunisierten Menschen beziehungsweise Tieres verstanden, wobei das Ziel darin besteht, antiinfektiöse oder antitoxische Antikörper in einen menschlichen oder tierischen Körper zur Vorbeugung oder Behandlung von Infektionskrankheiten zu übertragen.

Erfindungsgemäß ist vorgesehen, dass der Impfstoff als Lyophilisat oder als wässrige kolloidale Lösung oder Suspension vorliegt. Der erfindungsgemäße Impfstoff kann zusätzlich mindestens ein Adjuvants enthalten.

Die Erfindung betrifft ebenfalls einen Kit zur Identifizierung des Zellwandproteins Rbr1 von *Candida*-Arten oder eines mit Candida verwandten pathogenen Organismus und/oder zum Nachweis der Virulenz von *Candida*-Zellen oder von Zellen eines Organismus, der mit *Candida* verwandt ist, umfassend mindestens einen Behälter mit einem Antikörper, der Rbr1 p, und nicht erfindungsgemäß Rbr2p oder Rbr3p oder ein Fragment davon spezifisch erkennt und daran bindet. In bevorzugter Ausführungsform kann der Kit zur in vitro-Identifizierung von Rbr1p, und nicht erfindungsgemäß Rbr2p oder Rbr3p und/oder zum in vitro-Nachweis der Virulenz von *Candida-*Zellen eingesetzt werden. Der Kit kann gegebenenfalls einen zweiten Behälter mit dem isolierten und gereinigten Zellwandprotein Rbr1p, Rbr2p und/oder Rbr3p von *C*. *albicans* umfassen. Das isolierte und gereinigte Zellwandprotein Rbr1p oder Rbr2p oder Rbr3p dient vor allem als Kontrolle.

Die vorliegende Erfindung betrifft ebenfalls die Verwendung
eines Nucleinsäuremoleküls, das ein zur Hyphenentwicklung eines pathogenen pilzlichen Organismus erforderliches Zellwandprotein codiert und ausgewählt ist aus der Gruppe bestehend aus:
a) einem Nucleinsäuremolekül mit einer SEQ ID Nr. 1 dargestellten Nucleotidsequenz,
b) einem Nucleinsäuremolekül mit einer Nucleotidsequenz, die ein Protein mit einer in SEQ ID Nr. 2 dargestellten Aminosäuresequenz codiert,
c) einem Nucleinsäuremolekül mit einer Nucleotidsequenz, die über ihre gesamte Länge eine Homologie von mindestens 80% zu einer Nucleotidsequenz eines der Nucleinsäuremoleküle a) oder b) zeigt,
d) einem Nucleinsäuremolekül mit einer Nucleotidsequenz, die zu einer Nucleotidsequenz eines der Nucleinsäuremoleküle von a) bis c) komplementär ist, und
e) einem Fragment eines in a) bis d) definierten Nucleinsäuremoleküls, das in einer Wirtszelle in Antisense-Orientierung zu einem Promotor die Expression eines Zellwandproteins eines pathogenen pilzlichen Organismus hemmen kann und mindestens 10 Nucleotide umfasst;
eines Vektors, der das Nucleinsäuremolekül enthält;
einer Wirtszelle, die den Vektor enthält;
eines Proteins, das eine Aminosäuresequenz aus SEQ ID Nr. 2 enthält, oder
eines Antikörpers, der spezifisch das Protein erkennt und daran bindet
zur Diagnose von Krankheiten eines menschlichen oder tierischen Organismus, die durch *Candida*-Arten oder pathogene pilzliche Organismen, die mit *Candida* verwandt sind, verursacht werden.

Die vorliegende Erfindung betrifft ebenfalls die Verwendung
eines Nucleinsäuremoleküls, das ein zur Hyphenentwicklung eines pathogenen pilzlichen Organismus erforderliches Zellwandprotein codiert und ausgewählt ist aus der Gruppe bestehend aus:
a) einem Nucleinsäuremolekül mit einer SEQ ID Nr. 1 dargestellten Nucleotidsequenz,
b) einem Nucleinsäuremolekül mit einer Nucleotidsequenz, die ein Protein mit einer in SEQ ID Nr. 2 dargestellten Aminosäuresequenz codiert,
c) einem Nucleinsäuremolekül mit einer Nucleotidsequenz, die über ihre gesamte Länge eine Homologie von mindestens 80% zu einer Nucleotidsequenz eines der Nucleinsäuremoleküle a) oder b) zeigt,
d) einem Nucleinsäuremolekül mit einer Nucleotidsequenz, die zu einer Nucleotidsequenz eines der Nucleinsäuremoleküle von a) bis c) komplementär ist, und
e) einem Fragment eines in a) bis d) definierten Nucleinsäuremoleküls, das in einer Wirtszelle in Antisense-Orientierung zu einem Promotor die Expression eines Zellwandproteins eines pathogenen pilzlichen Organismus hemmen kann und mindestens 10 Nucleotide umfasst;
eines Vektors, der das Nucleinsäuremolekül enthält;
einer Wirtszelle, die den Vektor enthält;
eines Proteins, das eine Aminosäuresequenz aus SEQ ID Nr. 2 enthält, oder
eines Antikörpers, der spezifisch das Protein erkennt und daran bindet
zur Herstellung einer diagnostischen Zusammensetzung zur Diagnose von Krankheiten eines menschlichen oder tierischen Organismus, die durch *Candida*-Arten oder pathogene pilzliche Organismen, die mit *Candida* verwandt sind, verursacht werden.

In einer weiteren bevorzugten Ausführungsform betrifft die vorliegende Erfindung ebenfalls die Verwendung
eines Nucleinsäuremoleküls, das ein zur Hyphenentwicklung eines pathogenen pilzlichen Organismus erforderliches Zellwandprotein codiert und ausgewählt ist aus der Gruppe bestehend aus:
a) einem Nucleinsäuremolekül mit einer SEQ ID Nr. 1 dargestellten Nucleotidsequenz,
b) einem Nucleinsäuremolekül mit einer Nucleotidsequenz, die ein Protein mit einer in SEQ ID Nr. 2 dargestellten Aminosäuresequenz codiert,
c) einem Nucleinsäuremolekül mit einer Nucleotidsequenz, die über ihre gesamte Länge eine Homologie von mindestens 80% zu einer Nucleotidsequenz eines der Nucleinsäuremoleküle a) oder b) zeigt,
d) einem Nucleinsäuremolekül mit einer Nucleotidsequenz, die zu einer Nucleotidsequenz eines der Nucleinsäuremoleküle von a) bis c) komplementär ist, und
e) einem Fragment eines in a) bis d) definierten Nucleinsäuremoleküls, das in einer Wirtszelle in Antisense-Orientierung zu einem Promotor die Expression eines Zellwandproteins eines pathogenen pilzlichen Organismus hemmen kann und mindestens 10 Nucleotide umfasst;
eines Vektors, der das Nucleinsäuremolekül enthält;
einer Wirtszelle, die den Vektor enthält;
eines Proteins, das eine Aminosäuresequenz aus SEQ ID Nr. 2 enthält, oder
eines Antikörpers, der spezifisch das Protein erkennt und daran bindet
als Wirkstoff zur Behandlung und/oder Prävention von Krankheiten eines menschlichen oder tierischen Organismus, die durch *Candida-*Arten oder pathogene pilzliche Organismen, die mit *Candida* verwandt sind, verursacht werden. Die vorliegende Erfindung betrifft ebenfalls die Verwendung
eines Nucleinsäuremoleküls, das ein zur Hyphenentwicklung eines pathogenen pilzlichen Organismus erforderliches Zellwandprotein codiert und ausgewählt ist aus der Gruppe bestehend aus:
a) einem Nucleinsäuremolekül mit einer SEQ ID Nr. 1 dargestellten Nucleotidsequenz,
b) einem Nucleinsäuremolekül mit einer Nucleotidsequenz, die ein Protein mit einer in SEQ ID Nr. 2 dargestellten Aminosäuresequenz codiert,
c) einem Nucleinsäuremolekül mit einer Nucleotidsequenz, die über ihre gesamte Länge eine Homologie von mindestens 80% zu einer Nucleotidsequenz eines der Nucleinsäuremoleküle a) oder b) zeigt,
d) einem Nucleinsäuremolekül mit einer Nucleotidsequenz, die zu einer Nucleotidsequenz eines der Nucleinsäuremoleküle von a) bis c) komplementär ist, und
e) einem Fragment eines in a) bis d) definierten Nucleinsäuremoleküls, das in einer Wirtszelle in Antisense-Orientierung zu einem Promotor die Expression eines Zellwandproteins eines pathogenen pilzlichen Organismus hemmen kann und mindestens 10 Nucleotide umfasst;
eines Vektors, der das Nucleinsäuremolekül enthält;
einer Wirtszelle, die den Vektor enthält;
eines Proteins, das eine Aminosäuresequenz aus SEQ ID Nr. 2 enthält, oder
eines Antikörpers, der spezifisch das Protein erkennt und daran bindet
als Wirkstoff zur Herstellung einer pharmazeutischen Zusammensetzung zur Behandlung und/oder Prävention von Krankheiten, die durch *Candida-*Arten oder damit verwandte pathogene pilzliche Organismen verursacht werden. Erfindungsgemäß ist das Agens ausgewählt aus der Gruppe bestehend aus
einem Nucleinsäuremolekül, das ein zur Hyphenentwicklung eines pathogenen pilzlichen Organismus erforderliches Zellwandprotein codiert und ausgewählt ist aus der Gruppe bestehend aus:
a) einem Nucleinsäuremolekül mit einer SEQ ID Nr. 1 dargestellten Nucleotidsequenz,
b) einem Nucleinsäuremolekül mit einer Nucleotidsequenz, die ein Protein mit einer in SEQ ID Nr. 2 dargestellten Aminosäuresequenz codiert,
c) einem Nucleinsäuremolekül mit einer Nucleotidsequenz, die über ihre gesamte Länge eine Homologie von mindestens 80% zu einer Nucleotidsequenz eines der Nucleinsäuremoleküle a) oder b) zeigt,
d) einem Nucleinsäuremolekül mit einer Nucleotidsequenz, die zu einer Nucleotidsequenz eines der Nucleinsäuremoleküle von a) bis c) komplementär ist, und
e) einem Fragment eines in a) bis d) definierten Nucleinsäuremoleküls, das in einer Wirtszelle in Antisense-Orientierung zu einem Promotor die Expression eines Zellwandproteins eines pathogenen pilzlichen Organismus hemmen kann und mindestens 10 Nucleotide umfasst;
einem Vektor, der das Nucleinsäuremolekül enthält;
einer Wirtszelle, die den Vektor enthält;
eines Proteins, das eine Aminosäuresequenz aus SEQ ID Nr. 2 enthält, und
einem Antikörper, der spezifisch das Protein erkennt und daran bindet.

Die vorliegende Erfindung betrifft ebenfalls die Verwendung
eines Nucleinsäuremoleküls, das ein zur Hyphenentwicklung eines pathogenen pilzlichen Organismus erforderliches Zellwandprotein codiert und ausgewählt ist aus der Gruppe bestehend aus:
a) einem Nucleinsäuremolekül mit einer SEQ ID Nr. 1 dargestellten Nucleotidsequenz,
b) einem Nucleinsäuremolekül mit einer Nucleotidsequenz, die ein Protein mit einer in SEQ ID Nr. 2 dargestellten Aminosäuresequenz codiert,
c) einem Nucleinsäuremolekül mit einer Nucleotidsequenz, die über ihre gesamte Länge eine Homologie von mindestens 80% zu einer Nucleotidsequenz eines der Nucleinsäuremoleküle a) oder b) zeigt,
d) einem Nucleinsäuremolekül mit einer Nucleotidsequenz, die zu einer Nucleotidsequenz eines der Nucleinsäuremoleküle von a) bis c) komplementär ist, und
e) einem Fragment eines in a) bis d) definierten Nucleinsäuremoleküls, das in einer Wirtszelle in Antisense-Orientierung zu einem Promotor die Expression eines Zellwandproteins eines pathogenen pilzlichen Organismus hemmen kann und mindestens 10 Nucleotide umfasst;
einem Vektor, der das Nucleinsäuremolekül enthält;
einer Wirtszelle, die den Vektor enthält;
eines Proteins, das eine Aminosäuresequenz aus SEQ ID Nr. 2 enthält, oder
eines Antikörpers, der spezifisch das Protein erkennt und daran bindet
zur Identifizierung und/oder zum Nachweis von Substanzen, die die Expression oder Aktivität von Rbr1p, und nicht erfindungsgemäß Rbr2p und/oder Rbr3p in einem pathogenen pilzlichen Organismus hemmen und als Wirkstoff zur Herstellung einer pharmazeutischen Zusammensetzung geeignet sind.

In einer weiteren bevorzugten Ausführungsform betrifft die vorliegende Erfindung die Verwendung des Nucleinsäuremoleküls mit einer der in SEQ ID Nr. 1, und nicht erfindungsgemäß SEQ ID Nr. 3 oder SEQ ID Nr. 5 dargestellten Nucleotidsequenz, eines Nucleinsäuremoleküls mit einer Nucleotidsequenz, die ein Protein mit einer der in SEQ ID Nr. 2, und nicht erfindungsgemäß SEQ ID Nr. 4 oder SEQ ID Nr. 6 dargestellten Aminosäuresequenzen codiert, oder eines Fragmentes eines dieser Nucleinsäuremoleküle zur Isolierung einer homologen Nucleinsäure, die das Rbr1p-Protein, und nicht erfindungsgemäß das Rbr2p-Protein oder das Rbr3p-Protein von *C*. *tropicalis, C. krusei, C. parapsilosis, C. guilliermondii, C. glabrata, C. dubliniensis, C. lusitaniae,* einer Trichosporon-Art, einer Blastoschi-

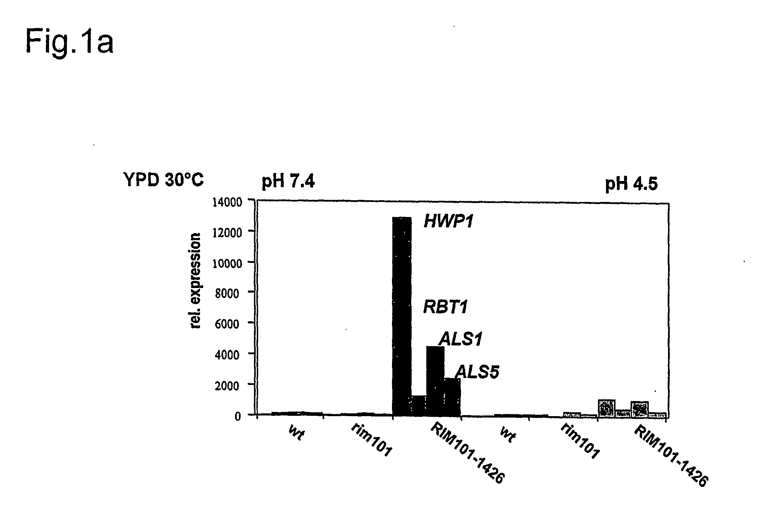

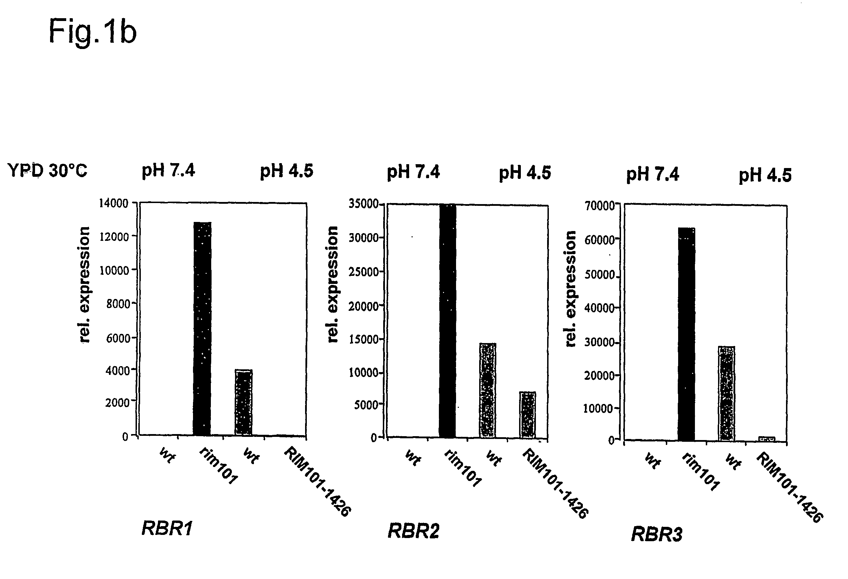

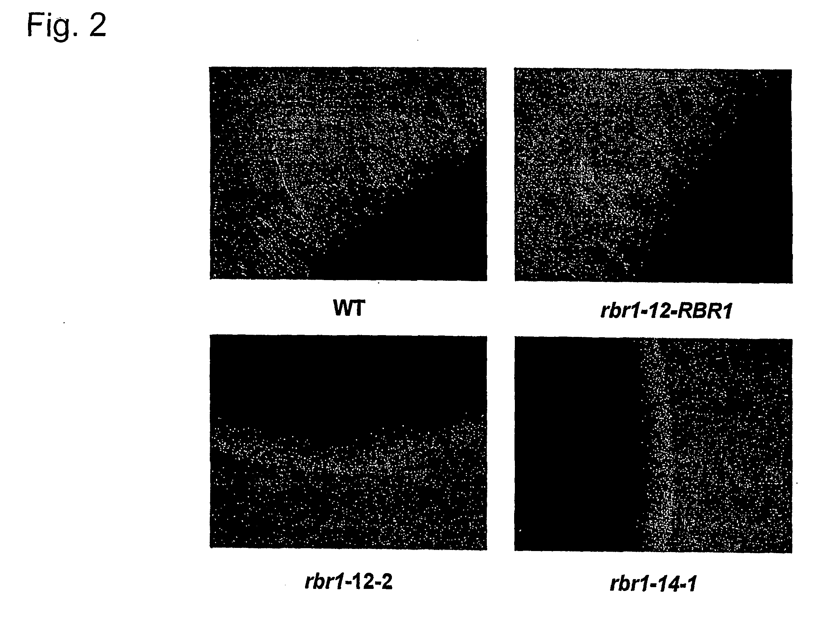

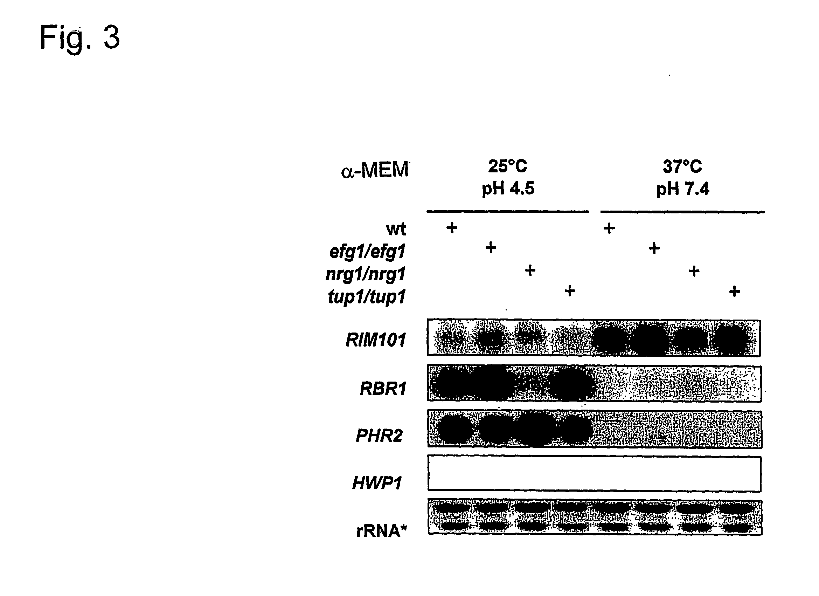

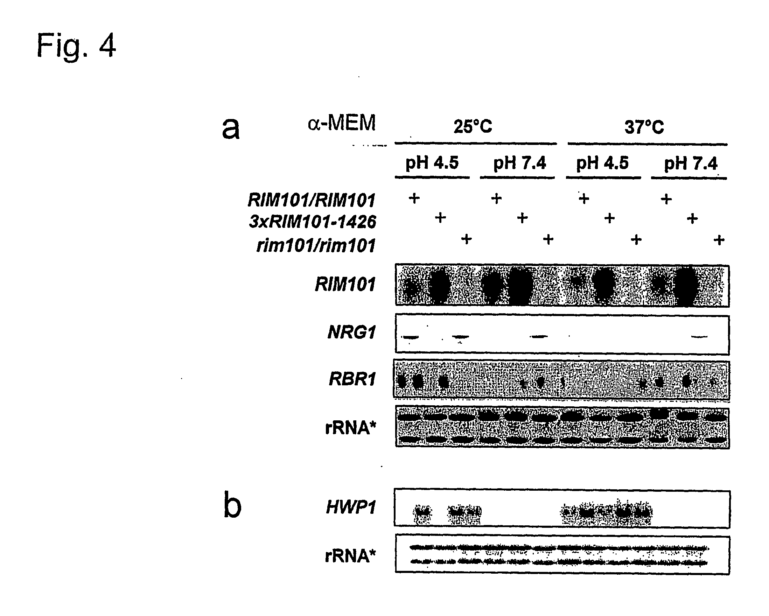

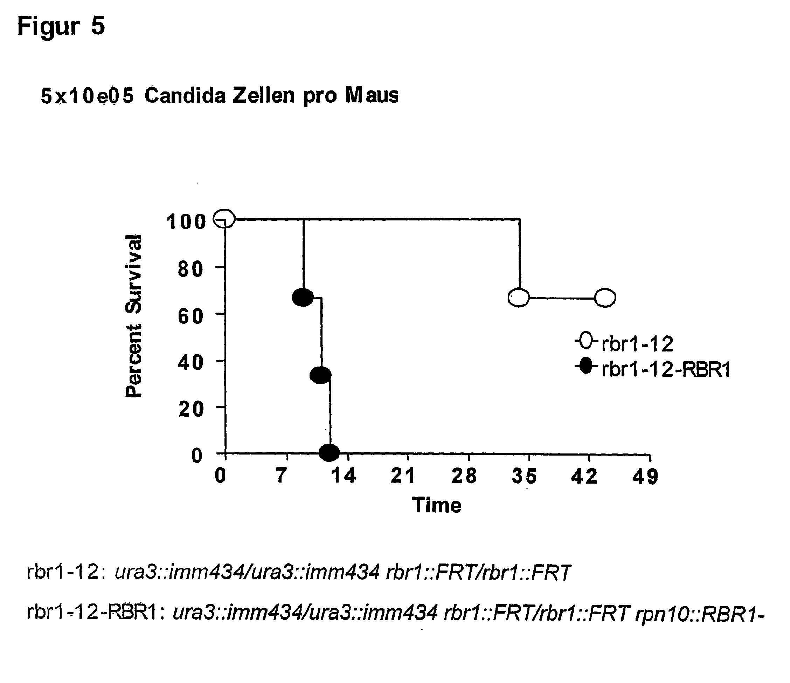

## Patentansprüche

1. *C*. *albicans*-Zelle enthaltend einen Vektor, worin ein Nucleinsäuremolekül, das ein zur Hyphenentwicklung eines pathogenen pilzlichen Organismus erforderliches Zellwandprotein codiert, in Antisense-Orientierung zu mindestens einem Regulationselement angeordnet ist und ausgewählt ist aus der Gruppe bestehend aus:
a) einem Nucleinsäuremolekül mit einer in SEQ ID Nr. 1 dargestellten Nucleotidsequenz,
b) einem Nucleinsäuremolekül mit einer Nucleotidsequenz, die ein Protein mit einer in SEQ ID Nr. 2 dargestellten Aminosäuresequenz codiert,
c) einem Nucleinsäuremolekül mit einer Nucleotidsequenz, die über ihre gesamte Länge eine Homologie von mindestens 80% zu einer Nucleotidsequenz eines der Nucleinsäuremoleküle von a) oder b) zeigt, und
d) einem Nucleinsäuremolekül mit einer Nucleotidsequenz, die zu einer Nucleotidsequenz eines der Nucleinsäuremoleküle von a) bis c) komplementär ist, und
e) einem Fragment eines in a) bis b) definierten Nucleinsäuremoleküls, das in einer Wirtszelle in Antisense-Orientierung zu einem Promotor die Expression eines Zellwandproteins eines pathogenen pilzlichen Organismus hemmen kann und mindestens 10 Nucleotide umfasst.

2. Verfahren zur Herstellung eines zur Hyphenentwicklung eines pathogenen pilzlichen Organismus erforderlichen Zellwandproteins, umfassend die Kultivierung einer Wirtszelle in einem geeigneten Kulturmedium unter Bedingungen, die eine Expression des Zellwandproteins erlauben, und die Gewinnung des exprimierten Zellwandproteins aus der Zelle oder aus dem Medium, **dadurch gekennzeichnet, dass** die Wirtszelle mindestens einen Vektor enthält, worin das in Anspruch 1 definierte Nucleinsäuremolekül in Antisense-Orientierung zu mindestens einem Regulationselement angeordnet ist.

3. Antikörper, der spezifisch ein Protein erkennt und daran bindet, **dadurch gekennzeichnet, dass** das Protein die Aminosäuresequenz SEQ ID Nr. 2 enthält, wobei der Antikörper spezifisch die Aminosäuresequenz SEQ ID Nr. 2 erkennt und daran bindet.

4. Antikörper nach Anspruch 3, wobei der Antikörper ein monoclonaler oder ein polyclonaler Antikörper ist.

5. Verfahren zur Charakterisierung und/oder zum Nachweis des Hyphenstadiums von *Candida*-Zellen oder Zellen pathogener pilzlicher Organismen einer *Trichosporon*-Art oder einer *Blastoschizomyces*-Art, umfassend die Inkubation der Zellen oder Zellfraktionen davon mit einem Mittel zur Identifizierung eines Zellwandproteins, das die Aminosäuresequenz SEQ ID Nr. 2 enthält, wobei der Nachweis des Proteins oder eines Fragmentes davon das Vorliegen des virulenten Hyphenstadiums der Zellen anzeigt.

6. Verfahren nach Anspruch 5, wobei die zu charakterisierenden *Candida-Zellen* Zellen von *C*. *albicans, C tropicalis, C*. *krusei, C*. *parapsilosis, C*. *guilliermondii, C*. *glabrata, C*. *dubliniensis* oder *C*. *lusitaniae* sind.

7. Verfahren nach Anspruch 5 oder 6, wobei die zu charakterisierenden Zellen in einer biologischen Probe vorliegen.

8. Verfahren nach einem der Ansprüche 5 bis 7, wobei die zu charakterisierenden Zellen aus einer biologischen Probe isolierte und angereicherte intakte Zellen sind.

9. Verfahren nach einem der Ansprüche 5 bis 8, wobei zur Charakterisierung isolierte Zellfraktionen eingesetzt werden, die durch Zellaufschluss und Fraktionierung von *Candida-Zellen* oder Zellen von mit *Candida* verwandten Arten erhältlich sind und mindestens eine Zellwand-Fraktion umfassen.

10. Verfahren nach einem der Ansprüche 5 bis 9, wobei das zur Identifizierung des Proteins eingesetzte Mittel ein immunologisches Mittel ist.

11. Verfahren nach Anspruch 10, wobei das immunologische Mittel ein gegen das Protein gerichtetes Antiserum, ein Antikörper nach Anspruch 3 oder 4 oder ein Fragment davon oder ein Komplex davon ist.

12. Verfahren nach Anspruch 11, wobei der Antikörper eine Markierung, ausgewählt aus der Gruppe bestehend aus einer Farbmarkierung, einer radioaktive Markierung, einer Fluoreszenzmarkierung, einer Chemilumineszenz-Markierung oder einem eine messbare Reaktion auslösenden Enzym, aufweist.

13. Verfahren zum Nachweis einer *Candida*-Infektion und/oder einer Infektion von pathogenen pilzlichen Organismen einer *Trichosporon-Art* oder einer *Blastoschizomyces-Art* in einer aus einem menschlichen oder tierischen Organismus erhaltenen biologischen Probe, wobei die Gegenwart des Zellwandproteins Rbr1p mit der SEQ ID Nr.2 und/oder eines Fragmentes davon in der biologischen Probe und/oder in der Zellwand von gegebenenfalls in der biologischen Probe enthaltenen *Candida-Zellen* oder Zellen von pathogenen pilzlichen Organismen einer *Trichosporon-Art* oder einer *Blastoschizomyces-Art* nachgewiesen wird, umfassend
a) die Inkubation der biologischen Probe mit einem Mittel zur Identifizierung des Zellwandproteins, das die Aminosäuresequenz SEQ ID Nr. 2 enthält, und
b) den Nachweis der Interaktion des Identifizierungsmittels mit dem Protein.

14. Verfahren nach Anspruch 13, wobei die *Candida-Zellen* Zellen von *C*. *albicans, C tropicalis, C. krusei, C. parapsilosis, C. guilliermondii, C. glabrata, C. dubliniensis* oder *C*. *lusitaniae* sind.

15. Verfahren nach Anspruch 13 oder 14, wobei die biologische Probe ein Haut- oder Schleimhautabstrich, eine Organbiopsie, eine Gewebebiopsie, eine Körperflüssigkeit, ein Körpersekret, Stuhl oder eine Spülung von Hohlräumen oder Hohlorganen ist.

16. Verfahren nach Anspruch 15, wobei die Körperflüssigkeit Sputum, Urin, Pleuraerguss, Liquor, Lymphe oder Blut ist.

17. Verfahren nach Anspruch 16, wobei das Blut als ungereinigte Blutprobe, Blutplasma oder Blutserum vorliegt.

18. Verfahren nach Anspruch 16 oder 17, wobei durch den Nachweis des Proteins in Blut oder in der Zellwand von im Blut enthaltenen *Candida*-Zellen invasive Candidiasis nachgewiesen wird.

19. Verfahren nach einem der Ansprüche 13 bis 18, wobei das zur Identifizierung des Proteins eingesetzte Mittel ein immunologisches Mittel ist.

20. Verfahren nach Anspruch 19, wobei das immunologische Mittel ein gegen das Protein gerichtetes Antiserum, ein Antikörper nach Anspruch 3 oder 4 oder ein Fragment davon oder ein Komplex davon ist.

21. Verfahren nach Anspruch 19 oder 20, wobei der Antikörper eine Markierung, ausgewählt aus der Gruppe bestehend aus einer Farbmarkierung, einer radioaktiven Markierung, einer Fluoreszenzmarkierung, einer Chemilumineszenz-Markierung oder einem eine messbare Reaktion auslösenden Enzym, aufweist.

22. Verfahren zum Auffinden und Identifizieren von Substanzen mit therapeutischer Wirkung gegen Krankheiten, die durch *Candida-*Arten oder pathogene pilzliche *Trichosporon-* oder *Blastoschizomyces*-Arten verursacht werden, wobei eine zu testende Substanz in einem geeigneten Medium mit mindestens einem Agens in Kontakt gebracht und eine Interaktion zwischen der zu testenden Substanz und dem Agens nachgewiesen wird, wobei das Agens ausgewählt ist aus der Gruppe bestehend aus:
einem Nucleinsäuremolekül, das ein zur Hyphenentwicklung eines pathogenen pilzlichen Organismus erforderliches Zellwandprotein codiert und ausgewählt ist aus der Gruppe bestehend aus:
a) einem Nucleinsäuremolekül mit einer in SEQ ID Nr. 1 dargestellten Nucleotidsequenz,
b) einem Nucleinsäuremolekül mit einer Nucleotidsequenz, die ein Protein mit einer in SEQ ID Nr. 2 dargestellten Aminosäuresequenz codiert,
c) einem Nucleinsäuremolekül mit einer Nucleotidsequenz, die über ihre gesamte Länge eine Homologie von mindestens 80% zu einer Nucleotidsequenz eines der Nucleinsäuremoleküle von a) oder b) zeigt,
d) einem Nucleinsäuremolekül mit einer Nucleotidsequenz, die zu einer Nucleotidsequenz eines der Nucleinsäuremoleküle von a) bis c) komplementär ist, und
e) einem Fragment eines in a) bis d) definierten Nucleinsäuremoleküls, das in einer Wirtszelle in Antisense-Orientierung zu einem Promotor die Expression eines Zellwandproteins eines pathogenen pilzlichen Organismus hemmen kann und mindestens 10 Nucleotide umfasst;
einem Vektor, der das Nucleinsäuremolekül enthält;
einer Wirtszelle, die den Vektor enthält;
einem Protein, das eine Aminosäuresequenz aus SEQ ID Nr. 2 enthält, und
einem Antikörper, der spezifisch das Protein erkennt und daran bindet.

23. Diagnostische Zusammensetzung, umfassend ein in Anspruch 22 genanntes Agens.

24. Pharmazeutische Zusammensetzung, umfassend ein in Anspruch 22 genanntes Agens.

25. Pharmazeutische Zusammensetzung nach Anspruch 24, wobei es sich um einen Impfstoff handelt, der ein in Anspruch 22 genanntes Protein enthält und der zur aktiven Immunisierung eines menschlichen oder tierischen Körpers gegen eine *Candida*-Infektion geeignet ist.

26. Pharmazeutische Zusammensetzung nach Anspruch 24, wobei es sich um einen Impfstoff handelt, der einen in Anspruch 22 genannten Antikörper enthält und der zur passiven Immunisierung eines menschlichen oder tierischen Körpers gegen eine *Candida-*Infektion geeignet ist.

27. Pharmazeutische Zusammensetzung nach Anspruch 24 oder 25, wobei der Impfstoff als Lyophilisat vorliegt.

28. Pharmazeutische Zusammensetzung nach Anspruch 24 oder 25, wobei der Impfstoff als wässrige kolloidale Lösung oder Suspension vorliegt.

29. Pharmazeutische Zusammensetzung nach einem der Ansprüche 25 bis 28, zusätzlich enthaltend mindestens ein Adjuvans.

30. Kit zur in vitro-Identifizierung des Zellwandproteins Rbr1p mit der SEQ ID Nr. 2 von *Candida-Arten* oder eines pathogenen Organismus einer *Trichosporon*-Art oder einer *Blastoschizomyces-Art* und/oder zum in vitro-Nachweis der Virulenz der Zellen, umfassend mindestens einen Behälter mit einem Antikörper nach Anspruch 3 oder 4.

31. Kit nach Anspruch 30, umfassend einen zweiten Behälter mit dem isolierten und gereinigten Protein mit einer in SEQ ID Nr. 2 dargestellten Aminosäuresequenz.

32. Verwendung eines in Anspruch 22 genannten Agens zur Diagnose von Krankheiten eines menschlichen oder tierischen Organismus, die durch *Candida-Arten* oder pathogene pilzliche Organismen einer *Trichosporon*-Art oder einer *Blastoschizomyces-Art* verursacht werden.

33. Verwendung eines in Anspruch 22 genannten Agens zur Herstellung einer diagnostischen Zusammensetzung zur Diagnose von Krankheiten eines menschlichen oder tierischen Organismus, die durch *Candida-Arten* oder pathogene pilzliche Organismen einer *Trichosporon*-Art oder einer *Blastoschizomyces-Art* verursacht werden.

34. Verwendung eines in Anspruch 22 genannten Agens als Wirkstoff zur Behandlung und/oder Prävention von Krankheiten eines menschlichen oder tierischen Organismus, die durch *Candida*-Arten oder pathogene pilzliche Organismen einer *Trichosporon*-Art oder einer *Blastoschizomyces-Art* verursacht werden.

35. Verwendung eines in Anspruch 22 genannten Agens als Wirkstoff zur Herstellung einer pharmazeutischen Zusammensetzung zur Behandlung und/oder Prävention von Krankheiten eines menschlichen oder tierischen Organismus, die durch *Candida*-Arten oder pathogene pilzliche Organismen einer *Trichosporon*-Art oder einer *Blastoschizomyces-Art* verursacht werden.

36. Verwendung eines in Anspruch 22 genannten Agens zur Identifizierung und/oder zum Nachweis von Substanzen, die die Expression oder Aktivität des Zellwandproteins Rbr1p mit der SEQ ID Nr.2 in einem pathogenen pilzlichen Organismus hemmen und als Wirkstoff zur Herstellung einer pharmazeutischen Zusammensetzung zur Bekämpfung von durch Candida-Arten verursachten Erkrankungen geeignet sind.

37. Verwendung eines Nucleinsäuremoleküls mit einer in SEQ ID Nr. 1 dargestellten Nucleotidsequenz, eines Nucleinsäuremoleküls mit einer Nucleotidsequenz, die ein Protein mit einer in SEQ ID Nr. 2 dargestellten Aminosäuresequenz codiert, oder eines Fragmentes davon zur Isolierung einer homologen Nucleinsäure, die das Rbr1 p-Zellwandprotein mit der SEQ ID Nr. 2 von *C*. *tropicalis, C. krusei, C. parapsilosis, C. guilliermondii, C. glabrata, C. dubliniensis, C. lusitaniae,* einer *Trichosporon-Art,* einer *Blastoschizomyces-Art* oder eines anderen pilzlichen pathogenen Organismus codiert.

38. Verwendung eines Antikörpers nach Anspruch 3 oder 4 zur Charakterisierung und/oder zum Nachweis des virulenten Hyphenstadiums von *Candida*-Zellen.

## Claims

1. A *C*. *albicans* cell containing a vector in which a nucleic acid molecule which encodes a cell wall protein necessary for the hyphae development of a pathogenic fungal organism is arranged in antisense orientation to at least one regulation element and is selected from the group consisting of:
a) a nucleic acid molecule having a nucleotide sequence as shown in SEQ ID No.1,
b) a nucleic acid molecule having a nucleotide sequence which encodes a protein having an amino acid sequence as shown in SEQ ID No. 2,
c) a nucleic acid molecule having a nucleotide sequence which shows a homology of at least 80% to a nucleotide sequence of one of the nucleic acid molecules of a) or b) over its entire length, and
d) a nucleic acid molecule having a nucleotide sequence which is complementary to a nucleotide sequence of one of the nucleic acid molecules of a) to c), and
e) a fragment of a nucleic acid molecule defined in a) to b) which can inhibit the expression of a cell wall protein of a pathogenic fungal organism in antisense orientation to a promoter in a host cell and comprises at least 10 nucleotides.

2. A method for the production of a cell wall protein necessary for the hyphae development of a pathogenic fungal organism, comprising the culturing of a host cell in a suitable culture medium under conditions which allow expression of the cell wall protein, and the obtainment of the expressed cell wall protein from the cell or from the medium, **characterized in that** the host cell contains at least one vector in which the nucleic acid molecule defined in claim 1 is arranged in antisense orientation to at least one regulation element.

3. An antibody which specifically recognizes a protein and binds thereto, **characterized in that** the protein contains the amino acid sequence SEQ ID No. 2, the antibody specifically recognizing the amino acid sequence SEQ ID No. 2 and binding thereto.

4. The antibody according to claim 3, the antibody being a monoclonal or a polyclonal antibody.

5. A method for the characterization and/or for the detection of the hyphae stage of *Candida* cells or cells of pathogenic fungal organisms of a *Trichosporon* species or of a *Blastoschizomyces* species, comprising the incubation of the cells or cell fractions thereof with an agent for the identification of a cell wall protein which contains the amino acid sequence SEQ ID No. 2, the detection of the protein or of a fragment thereof indicating the presence of the virulent hyphae stage of the cells.

6. The method according to claim 5, the *Candida* cells to be characterized being cells of *C*. *albicans, C*. *tropicalis, C. krusei, C. parapsilosis, C. guilliermondii, C. glabrata, C. dubliniensis* or *C*. *lusitaniae.*

7. The method according to claim 5 or 6, the cells to be characterized being present in a biological sample.

8. The method according to any one of claims 5 to 7, the cells to be characterized being cells isolated from a biological sample and enriched intact cells.

9. The method according to any one of claims 5 to 8, isolated cell fractions being employed for the characterization which are obtainable by cell disruption and fractionation of *Candida* cells or cells of species related to *Candida* and comprise at least one cell wall fraction.

10. The method according to any one of claims 5 to 9, the agent employed for the identification of the protein being an immunological agent.

11. The method according to claim 10, the immunological agent being an antiserum directed against the protein, an antibody according to claim 3 or 4 or a fragment thereof or a complex thereof.

12. The method according to claim 11, the antibody having a label selected from the group consisting of a dye label, a radiolabel, a fluorescent label, a chemiluminescent label or an enzyme inducing a measurable reaction.

13. A method for the detection of a *Candida* infection and/or of an infection by pathogenic fungal organisms of a *Trichosporon* species or of a *Blastoschizomyces* species in a biological sample obtained from a human or animal organism, the presence of the protein Rbr1p with SEQ ID No. 2 and/or of a fragment thereof in the biological sample and/or in the cell wall of *Candida* cells or cells of pathogenic fungal organisms of a *Trichosporon* species or of a *Blastoschizomyces* species optionally contained in the biological sample being detected, comprising
a) the incubation of the biological sample with an agent for the identification of the cell wall protein which contains the amino acid sequence SEQ ID No. 2, and
b) the detection of the interaction of the identification agent with the protein.

14. The method according to claim 13, the *Candida* cells being cells of *C. albicans, C. tropicalis, C. krusei, C. parapsilosis, C. guilliermondii, C. glabrata, C. dubliniensis* or *C*. *lusitaniae.*

15. The method according to claim 13 or 14, the biological sample being a skin or mucous membrane swab, an organ biopsy, a tissue biopsy, a body fluid, a body secretion, stool or a rinse from cavities or hollow organs.

16. The method according to claim 15, the body fluid being sputum, urine, pleural effusion, spinal fluid, lymph or blood.

17. The method according to claim 16, the blood being present as an unpurified blood sample, blood plasma or blood serum.

18. The method according to claim 16 or 17, invasive candidiasis being detected by the detection of the protein in blood or in the cell wall of Candida cells contained in the blood.

19. The method according to one of claims 13 to 18, the agent employed for the identification of the protein being an immunological agent.

20. The method according to claim 19, the immunological agent being an antiserum directed against the protein, an antibody according to claim 3 or 4 or a fragment thereof or a complex thereof.

21. The method according to claim 19 or 20, the antibody having a label selected from the group consisting of a dye label, a radiolabel, a fluorescent label, a chemiluminescent label or an enzyme inducing a measurable reaction.

22. A method for the discovery and identification of substances having therapeutic action against diseases which are caused by Candida species or pathogenic fungal Trichosporon or Blastoschizomyces species, a substance to be tested being brought into contact in a suitable medium with at least one agent and an interaction between the substance to be tested and the agent being detected, the agent being selected from the group consisting of:
a nucleic acid molecule which encodes a cell wall protein necessary for the hyphae development of a pathogenic fungal organism and is selected from the group consisting of:
a) a nucleic acid molecule having a nucleotide sequence as shown in SEQ ID No. 1,
b) a nucleic acid molecule having a nucleotide sequence which encodes a protein having an amino acid sequence as shown in SEQ ID No. 2,
c) a nucleic acid molecule having a nucleotide sequence which shows a homology of at least 80% to a nucleotide sequence of one of the nucleic acid molecules of a) or b) over its entire length,
d) a nucleic acid molecule having a nucleotide sequence which is complementary to a nucleotide sequence of one of the nucleic acid molecules of a) to c), and
e) a fragment of a nucleic acid molecule defined in a) to d) which can inhibit the expression of a cell wall protein of a pathogenic fungal organism in antisense orientation to a promoter in a host cell and comprises at least 10 nucleotides,
a vector which contains the nucleic acid molecule,
a host cell which contains the vector,
a protein which contains an amino acid sequence selected from SEQ ID No. 2, and
an antibody which specifically recognizes the protein and binds thereto.

23. A diagnostic composition comprising an agent **characterized in** claim 22.

24. A pharmaceutical composition comprising an agent **characterized in** claim 22.

25. The pharmaceutical composition according to claim 24, it being a vaccine which contains a protein **characterized in** claim 22 and which is suitable for the active immunization of a human or animal body against a *Candida* infection.

26. The pharmaceutical composition according to claim 24, it being a vaccine which contains an antibody **characterized in** claim 22 and which is suitable for the passive immunization of a human or animal body against a *Candida* infection.

27. The pharmaceutical composition according to in claim 24 or 25, the vaccine being present as a lyophilizate.

28. The pharmaceutical composition according to claim 24 or 25, the vaccine being present as an aqueous colloidal solution or suspension.

29. The pharmaceutical composition according to one of claims 25 to 28, additionally containing at least one adjuvant.

30. A kit for the in vitro identification of the cell wall protein Rbr1p with SEQ ID No. 2 of *Candida* species or of a pathogenic organism of a *Trichosporon* species or of a *Blastoschizomyces* species and/or for the in vitro detection of the virulence of the cells, comprising at least one container having an antibody according to claim 3 or 4.

31. The kit according to claim 30, comprising a second container having the isolated and purified protein having an amino acid sequence shown in SEQ ID No. 2.

32. Use of an agent **characterized in** claim 22 for the diagnosis of diseases of a human or animal organism which are caused by *Candida* species or pathogenic fungal organisms of a *Trichosporon* species or of a *Blastoschizomyces* species.

33. Use of an agent **characterized in** claim 22 for the production of a diagnostic composition for the diagnosis of diseases of a human or animal organism which are caused by *Candida* species or pathogenic fungal organisms of a *Trichosporon* species or of a *Blastoschizomyces* species.

34. Use of an agent **characterized in** claim 22 as an active compound for the treatment and/or prevention of diseases of a human or animal organism which are caused by *Candida* species or pathogenic fungal organisms of a *Trichosporon* species or of a *Blastoschizomyces* species.

35. Use of an agent **characterized in** claim 22 as an active compound for the production of a pharmaceutical composition for the treatment and/or prevention of diseases of a human or animal organism which are caused by *Candida* species or pathogenic fungal organisms of a *Trichosporon* species or of a *Blastoschizomyces* species.

36. Use of an agent **characterized in** claim 22 for the identification and/or for the detection of substances which inhibit the expression or activity of the cell wall protein Rbr1p with SEQ ID No. 2 in a pathogenic fungal organism and are suitable as an active compound for the production of a pharmaceutical composition for the control of diseases caused by *Candida* species.

37. Use of a nucleic acid molecule haying a nucleotide sequence shown in SEQ ID No. 1, of a nucleic acid molecule having a nucleotide sequence which encodes a protein having an amino acid sequence shown in SEQ ID No. 2, or of a fragment thereof for the isolation of a homologous nucleic acid which encodes the cell wall protein Rbr1p with SEQ ID No. 2 of *C*. *tropicalis, C. krusei, C. parapsilosis, C. guilliermondii, C. glabrata, C. dubliniensis, C. lusitaniae,* of a *Trichosporon* species, of a Blastoschizomyces species or of another fungal pathogenic organism.

38. Use of an antibody according to claim 3 or 4 for the characterization and/or for the detection of the virulent hyphae stage of *Candida* cells.

## Revendications

1. Cellule *C*. *albicans* comportant un vecteur, dans laquelle une molécule d'acide nucléique codant une protéine de membrane cellulaire nécessaire au développement des hyphes d'un organisme fongique pathogène est disposée dans le sens contraire à au moins un élément de régulation et sélectionnée dans le groupe consistant en :
a) une molécule d'acide nucléique présentant une séquence de nucléotides représentée dans SEQ ID No. 1,
b) une molécule d'acide nucléique présentant une séquence de nucléotides qui code une protéine avec une séquence d'acides aminés représentée dans SEQ ID No. 2,
c) une molécule d'acide nucléique présentant une séquence de nucléotides qui présente sur sa longueur totale une homologie d'au moins 80 % à une séquence de nucléotides d'une des molécules d'acide nucléique de a) ou b), et
d) une molécule d'acide nucléique présentant une séquence de nucléotides qui est complémentaire à une séquence de nucléotides d'une des molécules d'acide nucléique de a) à c), et
e) un fragment d'une molécule d'acide nucléique définie en a) à b) qui peut inhiber dans une cellule hôte dans le sens contraire à un promoteur l'expression d'une protéine de membrane cellulaire d'un organisme fongique pathogène et comprend au moins 10 nucléotides.

2. Procédé de fabrication d'une protéine de membrane cellulaire nécessaire au développement des hyphes d'un organisme fongique pathogène, comprenant la culture d'une cellule hôte dans un milieu de culture adapté dans des conditions propices à une expression de la protéine de membrane cellulaire, et l'obtention de la protéine de membrane cellulaire exprimée à partir de la cellule ou du milieu, **caractérisé en ce que** la cellule hôte comporte au moins un vecteur, dans lequel la molécule d'acide nucléique définie dans la revendication 1 est disposée dans le sens contraire à au moins un élément de régulation.

3. Anticorps, lequel détecte spécifiquement une protéine et s'y lie, **caractérisé en ce que** la protéine contient la séquence d'acides aminés SEQ ID No. 2, l'anticorps détectant spécifiquement la séquence d'acides aminés SEQ ID No. 2 et s'y liant.

4. Anticorps selon la revendication 3, l'anticorps étant un anticorps monoclonal ou polyclonal.

5. Procédé de caractérisation et/ou de mise en évidence du stade des hyphes de cellules *Candida* ou cellules d'organismes fongiques pathogènes d'une espèce *Trichosporon* ou d'une espèce *Blastoschizomyces,* comprenant l'incubation des cellules ou fractions cellulaires de celles-ci avec un moyen d'identification d'une protéine de membrane cellulaire contenant la séquence d'acides aminés SEQ ID No. 2, la mise en évidence de la protéine ou d'un fragment de celle-ci indiquant la présence du stade des hyphes virulent des cellules.

6. Procédé selon la revendication 5, les cellules *Candida* à caractériser étant des cellules de *C*. *albicans, C. tropicalis, C. krusei, C. parapsilosis, C. guilliermondii, C. glabrata, C. dubliniensis* ou *C*. *lusitaniae.*

7. Procédé selon la revendication 5 ou 6, les cellules à caractériser se trouvant dans un échantillon biologique.

8. Procédé selon l'une des revendications 5 à 7, les cellules à caractériser étant des cellules isolées à partir d'un échantillon biologique et enrichies intactes.

9. Procédé selon l'une des revendications 5 à 8, des fractions cellulaires isolées étant utilisées pour la caractérisation, lesquelles peuvent être obtenues par désintégration cellulaire et fractionnement de cellules *Candida* ou cellules d'espèces apparentées à *Candida* et comprennent au moins une fraction de membrane cellulaire.

10. Procédé selon l'une des revendications 5 à 9, le moyen utilisé pour identifier la protéine étant un moyen immunologique.

11. Procédé selon la revendication 10, le moyen immunologique étant un antisérum dirigé contre la protéine, un anticorps selon la revendication 3 ou 4 ou un fragment de celui-ci ou un complexe de celui-ci.

12. Procédé selon la revendication 11, l'anticorps présentant un marquage sélectionné dans le groupe consistant en un marquage de couleur, un marquage radioactif, un marquage fluorescent, un marquage par chimiluminescence ou une enzyme déclenchant une réaction mesurable.

13. Procédé de mise en évidence d'une infection par *Candida* et/ou d'une infection par des organismes fongiques pathogènes d'une espèce *Trichosporon* ou d'une espèce *Blastoschizomyces* dans un échantillon biologique obtenu à partir d'un organisme humain ou animal, la présence de la protéine de membrane cellulaire Rbr1p avec la SEQ ID No. 2 et/ou un fragment de celle-ci étant mise en évidence dans l'échantillon biologique et/ou dans la membrane cellulaire, selon le cas, des cellules *Candida* contenues dans l'échantillon biologique ou des cellules d'organismes fongiques pathogènes d'une espèce *Trichosporon* ou d'une espèce *Blastoschizomyces,* comprenant
a) l'incubation de l'échantillon biologique avec un moyen d'identification de la protéine de membrane cellulaire qui contient la séquence d'acides aminés SEQ ID No. 2, et
b) la mise en évidence de l'interaction du moyen d'identification avec la protéine.

14. Procédé selon la revendication 13, les cellules *Candida* étant des cellules de *C*. *albicans, C. tropicalis, C. krusei, C. parapsilosis, C. guilliermondii, C. glabrata, C. dubliniensis ou C. lusitaniae.*

15. Procédé selon la revendication 13 ou 14, l'échantillon biologique étant un prélèvement de peau ou de muqueuse, une biopsie d'organe, une biopsie de tissu, un fluide corporel, une sécrétion corporelle, des selles ou un fluide de lavage de cavités ou d'organes creux.

16. Procédé selon la revendication 15, le fluide corporel étant des expectorations, de l'urine, un épanchement pleural, du liquide cérébro-spinal, de la lymphe ou du sang.

17. Procédé selon la revendication 16, le sang étant sous forme d'échantillon de sang non nettoyé, de plasma sanguin ou de sérum sanguin.

18. Procédé selon la revendication 16 ou 17, une candidose invasive étant mise en évidence par la mise en évidence de la protéine dans le sang ou dans la membrane cellulaire par des cellules *Candida* contenues dans le sang.

19. Procédé selon l'une des revendications 13 à 18, le moyen utilisé pour identifier la protéine étant un moyen immunologique.

20. Procédé selon la revendication 19, le moyen immunologique étant un antisérum dirigé contre la protéine, un anticorps selon la revendication 3 ou 4 ou un fragment de celui-ci ou un complexe de celui-ci.

21. Procédé selon la revendication 19 ou 20, l'anticorps présentant un marquage sélectionné dans le groupe consistant en un marquage de couleur, un marquage radioactif, un marquage fluorescent, un marquage par chimiluminescence ou une enzyme déclenchant une réaction mesurable.

22. Procédé de détection et d'identification de substances ayant une action thérapeutique contre des maladies provoquées par des espèces *Candida* ou des espèces *Trichosporon* ou *Blastoschizomyces* fongiques pathogènes, une substance à tester étant mise en contact avec au moins un agent dans un milieu adapté et une interaction étant mise en évidence entre la substance à tester et l'agent, l'agent étant sélectionné dans le groupe consistant en :
une molécule d'acide nucléique codant une protéine de membrane cellulaire nécessaire au développement des hyphes d'un organisme fongique pathogène et sélectionnée dans le groupe consistant en :
a) une molécule d'acide nucléique présentant une séquence de nucléotides représentée dans SEQ ID No. 1,
b) une molécule d'acide nucléique présentant une séquence de nucléotides qui code une protéine avec une séquence d'acides aminés représentée dans SEQ ID No. 2,
c) une molécule d'acide nucléique présentant une séquence de nucléotides qui présente sur sa longueur totale une homologie d'au moins 80 % à une séquence de nucléotides d'une des molécules d'acide nucléique de a) ou b), et
d) une molécule d'acide nucléique présentant une séquence de nucléotides qui est complémentaire à une séquence de nucléotides d'une des molécules d'acide nucléique de a) à c), et
e) un fragment d'une molécule d'acide nucléique définie en a) à d) qui peut inhiber dans une cellule hôte dans le sens contraire à un promoteur l'expression d'une protéine de membrane cellulaire d'un organisme fongique pathogène et comprend au moins 10 nucléotides,
un vecteur qui contient la molécule d'acide nucléique,
une cellule hôte qui contient le vecteur,
une protéine qui contient une séquence d'acides aminés de SEQ ID No. 2, et
un anticorps qui détecte spécifiquement la protéine et s'y lie.

23. Composition de diagnostic comprenant un agent cité dans la revendication 22.

24. Composition pharmaceutique comprenant un agent cité dans la revendication 22.

25. Composition pharmaceutique selon la revendication 24, celle-ci s'agissant d'un vaccin qui contient une protéine citée dans la revendication 22 et convient à l'immunisation active d'un corps humain ou animal contre une infection par *Candida.*

26. Composition pharmaceutique selon la revendication 24, celle-ci s'agissant d'un vaccin qui contient un anticorps cité dans la revendication 22 et convient à l'immunisation passive d'un corps humain ou animal contre une infection par *Candida.*

27. Composition pharmaceutique selon la revendication 24 ou 25, le vaccin étant sous forme de lyophilisat.

28. Composition pharmaceutique selon la revendication 24 ou 25, le vaccin étant sous forme de solution colloïdale aqueuse ou suspension.

29. Composition pharmaceutique selon l'une des revendications 25 à 28, contenant en outre au moins un adjuvant.

30. Kit d'identification *in vitro* de la protéine de membrane cellulaire Rbr1p avec la SEQ ID No. 2 d'espèces *Candida* ou d'un organisme pathogène d'une espèce *Trichosporon* ou d'une espèce *Blastoschizomyces* et/ou de mise en évidence *in vitro* de la virulence des cellules, comprenant au moins un récipient avec un anticorps selon la revendication 3 ou 4.

31. Kit selon la revendication 30, comprenant un second récipient avec la protéine isolée et nettoyée présentant une séquence d'acides aminés représentée dans SEQ ID No. 2.

32. Utilisation d'un agent cité dans la revendication 22 pour diagnostiquer des maladies affectant un organisme humain ou animal qui sont provoquées par des espèces *Candida* ou organismes fongiques pathogènes d'une espèce *Trichosporon* ou d'une espèce *Blastoschizomyces.*

33. Utilisation d'un agent cité dans la revendication 22 pour fabriquer une composition de diagnostic destinée à diagnostiquer des maladies affectant un organisme humain ou animal qui sont provoquées par des espèces *Candida* ou organismes fongiques pathogènes d'une espèce *Trichosporon* ou d'une espèce *Blastoschizomyces.*

34. Utilisation d'un agent cité dans la revendication 22 comme substance active pour le traitement et/ou la prévention de maladies affectant un organisme humain ou animal qui sont provoquées par des espèces *Candida* ou organismes fongiques pathogènes d'une espèce *Trichosporon* ou d'une espèce *Blastoschizomyces.*

35. Utilisation d'un agent cité dans la revendication 22 comme substance active pour la fabrication d'une composition pharmaceutique servant à traiter et/ou prévenir des maladies affectant un organisme humain ou animal qui sont provoquées par des espèces *Candida* ou organismes fongiques pathogènes d'une espèce *Trichosporon* ou d'une espèce *Blastoschizomyces.*

36. Utilisation d'un agent cité dans la revendication 22 pour identifier et/ou mettre en évidence des substances qui inhibent l'expression ou l'activité de la protéine de membrane cellulaire Rbr1p avec la SEQ ID No. 2 dans un organisme fongique pathogène et conviennent pour servir de substance active pour la fabrication d'une composition pharmaceutique destinée à lutter contre des affections provoquées par des espèces *Candida.*

37. Utilisation d'une molécule d'acide nucléique avec une séquence de nucléotides représentée dans SEQ ID No. 1, d'une molécule d'acide nucléique avec une séquence de nucléotides qui code une protéine avec la séquence d'acides aminés représentée dans SEQ ID No. 2, ou d'un fragment de celle-ci pour isoler un acide nucléique homologue qui code la protéine de membrane cellulaire Rbr1p avec la SEQ ID No. 2 de *C*. *tropicalis, C. krusei, C. parapsilosis, C. guilliennondii, C. glabrata, C. dubliniensis, C. lusitaniae,* d'une espèce *Trichosporon,* d'une espèce *Blastoschizomyces* ou d'un autre organisme pathogène fongique.

38. Utilisation d'un anticorps selon la revendication 3 ou 4 pour la caractérisation et/ou la mise en évidence du stade des hyphes virulent de cellules *Candida.*
